# EUROPEAN PATENT APPLICATION

(11) **EP 4 793 961 A1**
(43) Date of publication of application: **19.08.2026**
(21) Application number: 25172051.2
(22) Date of filing: 23.04.2025
(51) Int. Cl.: G16H 20/00, G06F 16/30, G06F 40/30, G06N 3/0455, G16H 20/30, G16H 20/70, G16H 70/20

(54) **STRUCTURED TREATMENT PLAN GENERATION AND CONTENT INTEGRATION IN DIGITAL THERAPEUTIC APPLICATIONS**

(30) Priority: 18.02.2025 US 202519056168
(71) Applicant: Click Therapeutics, Inc., New York, NY 10013 (US)
(72) Inventor: Qiu, Chuanhan, New York, 10013 (US); Djmal, Ary, New York, 10013 (US)
(74) Representative: Marks & Clerk LLP

(57) **Abstract**

Various examples, systems, and/or methods are disclosed relating to a digital therapeutic development process and pipeline. One or more processors can process natural language treatment inputs, generate a structured treatment plan for use in a digital therapeutics application, retrieve content from a database to align with the structured treatment plan, and arrange the content in a design system according to the structured treatment plan.

## Description

### CROSS REFERENCES TO RELATED APPLICATIONS

The present application claims the benefit of and priority to U.S. Non-Provisional Application No. 19/056,168, titled "STRUCTURED TREATMENT PLAN GENERATION AND CONTENT INTEGRATION IN DIGITAL THERAPEUTIC APPLICATIONS," filed on February 18, 2025, which is incorporated herein by reference in entirety.

### BACKGROUND

Treatment planning for health conditions, including mental health disorders, chronic diseases, and/or rehabilitative therapies, often involves structuring multi-step interventions, exercises, and/or condition-specific therapeutic workflows. Common conditions for structured treatment planning include post-traumatic stress disorder (PTSD), substance use disorders, cardiovascular diseases, obesity, migraines, multiple sclerosis, oncology-related conditions, and/or any other acute or chronic medical or psychological conditions. Treatment planning challenges arise due to a variety of factors, such as variability in user needs, differences in provider approaches, limitations in existing digital therapeutic tools, and/or a lack of automation in treatment plan generation. For example, factors that contribute to these challenges include non-standardized treatment methodologies (e.g., differences in treatment plan component sequencing, variations in prescribed therapeutic activities), insufficient personalization (e.g., failure to account for user-specific symptoms, comorbidities, and treatment responsiveness), and/or accessibility barriers (e.g., limited provider availability, constraints in delivering therapy across diverse populations). Other factors include inefficiencies in manual treatment plan construction (e.g., time-intensive development of structured treatment plans), a lack of integration between treatment planning tools and digital therapeutic applications, and/or difficulties in adapting existing treatment plans to real-time user data.

The effectiveness and/or efficacy of treatment plans is often compromised by limitations in structure, adaptability, and accessibility. Users receiving treatment plans that are not structured to their individual progress, symptom severity, or engagement levels can experience delayed therapeutic outcomes, reduced adherence, increased frustration, and/or diminished motivation to complete components of the treatment plans. Additionally, healthcare providers and digital therapeutic developers face challenges in constructing, modifying, and/or implementing structured treatment journeys, which limits the scalability of personalized therapy. Users engaging with rigid and/or non-adaptive treatment plans are at a greater risk of encountering difficulties in sustaining engagement, progressing through therapeutic exercises, responding to condition-specific triggers, integrating treatment recommendations into daily life, and/or adhering to long-term health strategies. The overall efficacy of structured treatment plans is often diminished due to these limitations.

At the technical level, treatment planning inefficiencies lead to challenges in designing structured execution frameworks that provide treatment plan sequencing, adapt to user progress, and/or integrate with digital therapeutic applications. Specifically, prior systems lack mechanisms to convert natural language treatment goals into structured execution frameworks, limiting the ability to streamline treatment plan delivery. Furthermore, users and healthcare providers relying on non-automated treatment planning systems face increased risks of misalignment between treatment goals and prescribed treatments (e.g., lack of conditional logic to adjust activities based on user progress), limited continuity of care across different health conditions (e.g., difficulties in integrating behavioral therapy with medical treatment plans), and/or reduced responsiveness to evolving user needs. For example, a manually designed plan can fail to incorporate dynamic feedback loops, resulting in fixed pathways that do not adjust to user-reported distress levels. In another example, a structured weight management program can lack adaptability based on real-time user-reported dietary adherence. In yet another example, a rehabilitation plan for post-surgical recovery cannot dynamically adjust exercise intensity based on functional progress assessments.

Conventional solutions suffer from a lack of automation, inefficient personalization mechanisms, and/or insufficient integration with digital ecosystems. In particular, the reliance on manual treatment plan generation, the limited ability of digital tools to structure adaptive execution frameworks, and/or the difficulty in integrating existing therapeutic modules into treatment workflows reduce the scalability and effectiveness of digital treatment planning. For example, prior workflow-based digital therapy solutions can require significant manual input from providers to define treatment paths, reducing efficiency in clinical settings. In another example, conventional treatment planning software can fail to use models to create structured, multi-step treatment journeys based on user-specific input. In yet another example, prior digital applications cannot dynamically adjust content presentation, therapeutic activities, or user interactions based on predefined scheduling parameters and conditional logic. Failing to incorporate automated, structured, and adaptive mechanisms into treatment planning solutions can result in suboptimal therapeutic engagement and diminished user outcomes.

### SUMMARY

Presented herein are systems and methods for generating structured treatment plans from natural language input using generative artificial intelligence (genAI) models. The system described herein relates to processing natural language treatment inputs, generating a structured treatment plan for use in a digital therapeutics application, retrieving content from a database to align with the structured treatment plan, and arranging the content in a design system according to the structured treatment plan, thus allowing for rapid iteration and deployment of a large number of personalized digital therapeutic applications, each individually tailored for the respective user. The generative models (e.g., models for treatment plan structuring, treatment component sequencing, and/or conditional logic generation) generate a structured representation of a treatment plan based on user-provided or physician-provided input. For example, the system can process a request such as "create a 3-month treatment journey for an individual with PTSD who has triggers in the afternoon, in the middle of the workday, and on days in the middle of the workweek that includes a schedule for breathing exercises, grounding exercises, interactive therapeutic intervention activities, medication administration, and the first five days of treatment based on a predefined template" and generate a treatment plan that defines the sequence of therapeutic actions to be performed by the user, retrieves corresponding content from a database, arranges content in a design system, and/or structures a treatment plan to support rendering within a digital therapeutic application personalized to the user.

Specifically, this improved approach facilitates automated structuring of a larger number of treatment plans, including dynamically generated execution logic, predefined therapeutic workflows, and modular content retrieval, which functions to improve treatment adherence, enhance engagement, and streamline intervention delivery. The system applies structured treatment goals to digital therapeutic applications, retrieving and structuring therapeutic components to present to a user such as smoking cessation task sequences based on predefined execution frameworks. The structured data package and/or structured execution framework provides a representation of the treatment plan, defining how therapeutic components are arranged, scheduled, and/or conditionally triggered within the digital therapeutic application.

The technical improvement can be obtained by performing (e.g., continuously, periodically, and/or based on user progress) treatment plan structuring, modular content retrieval, and/or adaptive therapeutic sequencing in a digital execution environment. The structured execution framework provides a structured representation of a treatment plan by defining the arrangement, scheduling, and conditional logic governing therapeutic components. The system generates the structured execution framework by processing a treatment plan, retrieving corresponding content from a database, and organizing the retrieved content into a structured data representation in a design system. The structured execution framework defines how therapeutic components are linked, scheduled, and/or conditionally presented within a digital therapeutic application. For example, the system can structure a treatment plan by associating retrieved content with scheduled treatment actions, specifying transition conditions as to when to present therapeutic activities, and defining execution dependencies to support structured intervention delivery.

The integration is a technical improvement over prior treatment planning techniques that rely on manual design, rigid therapy schedules, and/or predefined treatment paths, the prior treatment planning techniques without automated structuring and/or adaptive progression mechanisms. If there is an imbalance between treatment structures and user-specific therapeutic needs, the structured execution framework and/or modular content retrieval mechanisms disclosed herein can improve personalization, adaptability, and/or efficiency in treatment plan generation. The combination of structured execution frameworks and content retrieval from a database facilitates scalable deployment of a large number of digital therapeutic treatment plans and/or automated adaptation of treatment strategies.

Additionally, the digital therapeutic application described herein addresses the lack of generated treatment structuring in prior approaches, by integrating real-time treatment plan generation, structured sequencing logic, and/or modular content retrieval. The system uses structured execution frameworks with predefined conditional logic to dynamically organize and/or arrange content retrieved from a database, ensuring that the treatment plan aligns with structured treatment workflows. The structured execution framework can incorporate structured rules for determining how therapeutic content is retrieved, scheduled, and/or presented within a digital therapeutic application.

Accordingly, the digital therapeutic application addresses inefficiencies in treatment planning by providing structured execution frameworks that define treatment progression, schedule therapeutic activities, and/or retrieve content based on structured queries to a database and incorporated into a design system. The structured execution framework provides a structured format for arranging, linking, and presenting therapeutic content in a digital therapeutic application. Through the integration of generative AI, structured data frameworks, and/or modular content retrieval, the system improves scalability and/or adaptability in the development of a large number of digital therapeutic applications, leading to improved structured treatment plan delivery and improved accessibility of treatment plans. Additionally, the digital therapeutic application system discussed herein addresses the challenge of creating a generative AI-based platform individually tailored for a large number of users needing treatment across a range of physical, cognitive, social, and/or behavioral domains.

Additionally, the systems and methods described herein incorporate structured execution models, conditional logic frameworks, and/or dynamic treatment plan generation techniques to improve efficiency in large-scale treatment plan structuring, reduce the complexity of manual design, and enhance integration with digital platforms globally (e.g., interoperability with electronic health record systems, wearable devices, and/or other products and devices that the system described herein could interact with). By providing structured, dynamically generated treatment pathways, the disclosed systems and methods improve consistency, structured content arrangement, and/or treatment workflow execution in digital therapeutic applications.

Some implementations relate to a system including one or more processors coupled with memory. The one or more processors configured to receive natural language input to generate a treatment plan for a digital therapeutic application to address a condition. The one or more processors configured to apply the natural language input and the condition as input to at least one generative model to cause the at least one generative model to generate a treatment plan for the condition. In some implementations, the treatment plan includes a list of treatment components and a schedule for presenting at least one treatment component of the list of treatment components. The one or more processors configured to retrieve, from a database a plurality of content items corresponding with at least one treatment component of the list of treatment components in the treatment plan. The one or more processors configured to arrange, using the plurality of content items and the schedule, the list of treatment components of the treatment plan into a structured data package for presentation in the digital therapeutic application. The one or more processors configured to provide the structured data package.

In some implementations, the one or more processors coupled with the memory is further configured to generate, using the structured data package, a plurality of instructions configured to cause the digital therapeutic application to present at least one of the plurality of content items according to the arrangement of the list of treatment components of the treatment plan.

In some implementations, the structured data package corresponds to a mapping of the plurality of content items, the schedule, and/or a plurality of execution dependencies of the structured data package.

In some implementations, the treatment plan includes the schedule identifying a plurality of conditional steps corresponding with at least one of the list of treatment components of a treatment journey.

In some implementations, the schedule of the treatment plan includes a plurality of conditional logic corresponding with at least one treatment component and initiating at least one subsequent treatment component. In some implementations, the at least one subsequent treatment component is provided based at least on a completion of or failure of at least one prior treatment component or a completion of or a failure to meet predefined criteria for the at least one prior treatment component.

In some implementations, the one or more processors coupled with the memory is further configured to compile skeleton code retrieved from at least one database or generate, using the structured data package. In some implementations, the skeleton code includes at least one programmatic construct corresponding with the list of treatment components of the treatment plan and the schedule.

In some implementations, the one or more processors coupled with the memory is further configured to provide the treatment plan or the skeleton code to an administrator for review. In some implementations, the one or more processors coupled with the memory is further configured to receive, from the administrator, at least one update to the treatment plan or the skeleton code prior to providing the structured data package or using the skeleton code in the digital therapeutic application.

Some implementations relate to a system including one or more processors coupled with memory. The one or more processors configured to receive natural language input to generate content for a digital therapeutic application to address a condition. The one or more processors configured to apply the natural language input and the condition as input to at least one generative model to cause the at least one generative model to generate a directed process graph for the condition, the directed process graph includes a plurality of actions and conditional logic according to which to provide, for presentation, at least one of the plurality of actions. The one or more processors configured to identify, at least one data object corresponding with at least one of the plurality of actions included in the directed process graph. The one or more processors configured to generate, using the at least one data object and the directed process graph, a structured execution framework identifying (i) a plurality of content items identifying at least one of the plurality of actions and (ii) corresponding conditional logic according to which to provide, for presentation, at least one of the plurality of actions. The one or more processors configured to provide the structured execution framework.

In some implementations, the one or more processors coupled with the memory is further configured to generate, using the structured execution framework, a plurality of instructions configured to cause the digital therapeutic application to present the content including at least one of the plurality of content items according to the directed process graph.

In some implementations, the structured execution framework corresponds to a mapping of the plurality of content items, the corresponding conditional logic, a plurality of timing parameters, and/or a plurality of execution dependencies of the directed process graph.

In some implementations, the one or more processors coupled with the memory is further configured to emulate, using an emulator, performance of the plurality of actions based on processing at least a portion of the plurality of instructions according to the structured execution framework. In some implementations, the plurality of instructions includes at least one operation for performing at least one of the plurality of actions corresponding with at least one timing parameter.

In some implementations, the directed process graph includes a hierarchical arrangement of components representing the plurality of actions, the hierarchical arrangement arranged based on the plurality of timing parameters identifying a sequence and duration corresponding with at least one of the plurality of actions.

In some implementations, the at least one generative model is at least one of (i) a deep learning model, (ii) a supervised learning model, and/or (iii) an unsupervised learning model. In some implementations, causing the at least one generative model to generate the directed process graph includes processing the natural language input to extract a plurality of actionable elements, associating the plurality of actionable elements with the plurality of actions and a plurality of conditional logic, and/or arranging the plurality of actions and the plurality of conditional logic into a hierarchical structure.

In some implementations, the conditional logic of the directed process graph includes a plurality of conditional logic corresponding with at least one treatment component and initiating at least one subsequent treatment component. In some implementations, the at least one subsequent treatment component is provided based at least on a completion of or failure of at least one prior treatment component or a completion of or a failure to meet predefined criteria for the at least one prior treatment component.

In some implementations, the natural language input includes instructions identifying at least one of (i) a treatment duration, (ii) at least one treatment component, (iii) at least one action corresponding to the at least one treatment component, and/or (iv) at least one criteria for completing the at least one treatment component.

In some implementations, the directed process graph includes a plurality of execution paths. In some implementations, the plurality of execution paths of the directed process graph correspond to a plurality of treatment workflows of a treatment journey. In some implementations, each treatment workflow of the plurality of treatment workflows includes a sequence of therapeutic actions to address or manage the condition.

In some implementations, at least one of the plurality of actions corresponds to a treatment component or a response to a diagnostic query. In some implementations, the one or more processors coupled with the memory is further configured to in response to a completion of the treatment component or providing the response to the diagnostic query, update an activity record to include a completion status, timestamp data, and/or data generated or obtained during the treatment component or diagnostic query.

In some implementations, the one or more processors coupled with the memory is further configured to compile skeleton code retrieved from at least one database or generate, using the structured execution framework and the directed process graph, the skeleton code including at least one programmatic construct corresponding with the plurality of actions and corresponding conditional logic.

In some implementations, the one or more processors coupled with the memory is further configured to provide the directed process graph or the skeleton code to an administrator for review. In some implementations, the one or more processors coupled with the memory is further configured to receive, from the administrator, at least one update to the directed process graph or the skeleton code prior to providing the structured execution framework or using the skeleton code in the digital therapeutic application.

In some implementations, identifying the at least one data object includes querying the database maintaining the at least one data object. In some implementations, querying the database includes obtaining, using at least one application programming interface (API) request, the at least one data object corresponding to at least one historical therapeutic component or configuration.

In some implementations, the at least one data object includes at least one of the plurality of content items and corresponding parameters. In some implementations, the corresponding parameters include at least one of a content item relationship, a timing parameter, and/or metadata for representing the at least one historical therapeutic component as a node or relationship in the directed process graph.

Some implementations relate to a method. The method includes receiving, by one or more processors, natural language input to generate content for a digital therapeutic application to address a condition. The method includes applying, by the one or more processors, the natural language input and the condition as input to at least one generative model to cause the at least one generative model to generate a directed process graph for the condition. In some implementations, the directed process graph includes a plurality of actions and conditional logic according to which to provide, for presentation, at least one of the plurality of actions. The method includes identifying, by the one or more processors, at least one data object corresponding with at least one of the plurality of actions included in the directed process graph. The method includes generating, by the one or more processors using the at least one data object and the directed process graph. In some implementations, the structured execution framework can identify (i) a plurality of content items identifying at least one of the plurality of actions and (ii) corresponding conditional logic according to which to provide, for presentation, at least one of the plurality of actions. The method includes providing, by the one or more processors, the structured execution framework.

In some implementations, the method includes generating, by the one or more processors using the structured execution framework, a plurality of instructions configured to cause the digital therapeutic application to present the content including at least one of the plurality of content items according to the directed process graph.

In some implementations, the structured execution framework corresponds to a mapping of the plurality of content items, the corresponding conditional logic, a plurality of timing parameters, and/or a plurality of execution dependencies of the directed process graph.

In some implementations, the method includes emulating, by one or more processors using an emulator, performance of the plurality of actions based on processing at least a portion of the plurality of instructions according to the structured execution framework. In some implementations, the plurality of instructions includes at least one operation for performing at least one of the plurality of actions corresponding with at least one timing parameter.

In some implementations, the at least one generative model is at least one of (i) a deep learning model, (ii) a supervised learning model, and/or (iii) an unsupervised learning model. In some implementations, causing the at least one generative model to generate the directed process graph includes processing the natural language input to extract a plurality of actionable elements, associating the plurality of actionable elements with the plurality of actions and a plurality of conditional logic, and/or arranging the plurality of actions and the plurality of conditional logic into a hierarchical structure.

In some implementations, the conditional logic of the directed process graph includes a plurality of conditional logic corresponding with at least one treatment component and initiating at least one subsequent treatment component. In some implementations, the at least one subsequent treatment component is provided based at least on a completion of or failure of at least one prior treatment component or a completion of or a failure to meet predefined criteria for the at least one prior treatment component.

In some implementations, the natural language input includes instructions identifying at least one of (i) a treatment duration, (ii) at least one treatment component, (iii) at least one action corresponding to the at least one treatment component, and/or (iv) at least one criteria for completing the at least one treatment component.

In some implementations, the directed process graph includes a plurality of execution paths. In some implementations, the plurality of execution paths of the directed process graph correspond to a plurality of treatment workflows of a treatment journey. In some implementations, each treatment workflow of the plurality of treatment workflows includes a sequence of therapeutic components to address or manage the condition.

### BRIEF DESCRIPTION OF THE DRAWINGS

The present systems and methods for structured treatment plan generation and content integration in digital therapeutic applications are described in detail below with reference to the attached drawing figures, wherein:
FIG. 1 is a block diagram of an example of a system, in accordance with some implementations of the present disclosure;
FIG. 2 is a flow diagram of an example method for generating a structured execution framework, in accordance with some implementations of the present disclosure;
FIGS. 3A-3B are example interfaces for interacting with a generative model to generate a structured execution framework, in accordance with some implementations of the present disclosure;
FIG. 4 is a block diagram of an example server system and an example administrator device in accordance with an illustrative embodiment, in accordance with some implementations of the present disclosure.

### DETAILED DESCRIPTION

This disclosure relates to systems and methods for generating structured treatment plans from natural language input, retrieving content from a database, and providing a structured execution framework or structured data package for use in a digital therapeutic application. For example, systems and methods in accordance with the present disclosure facilitate the curation and modeling of structured treatment workflows by processing natural language and/or free-text descriptions, generating directed process graphs, and associating retrieved content with structured therapeutic treatment plans. The systems can receive a natural language input describing a treatment goal, apply the input to at least one generative artificial intelligence (AI) model configured to generate a directed process graph, and retrieve corresponding content items from a database to generate a structured execution framework. The structured execution framework can define the arrangement, scheduling, and/or conditional logic governing therapeutic components for rendering within a digital therapeutic application.

Some conventional treatment planning techniques rely on manually curated treatment module sequences, rigid therapy schedules, and/or predefined activity paths that lack flexibility in structuring and organizing treatment plans. These prior approaches fail to establish treatment progression with structured execution rules or define flexible relationships between therapeutic workflows and content sources. Additionally, such conventional methods lack integrated mechanisms for structuring and retrieving treatment content based on predefined treatment plan models, resulting in inefficiencies in digital therapeutic application development. For example, prior systems cannot define structured treatment plans, map retrieved content to structured treatment plan workflows, and/or model execution frameworks to establish therapy sequencing. The systems and methods described herein overcome these limitations by generating and/or otherwise modeling digital therapeutic treatment plans using various models and/or functions, which structure outputs based on natural language input, retrieved content, and predefined treatment parameters.

Systems and methods in accordance with the present disclosure include receiving, during a process, natural language input from an administrator device corresponding to a treatment goal for a condition. For example, the system can apply the natural language input to at least one AI model to generate a directed process graph, defining a plurality of actions and corresponding conditional logic. The system can identify at least one data object corresponding to at least one of the plurality of actions and retrieve content items associated with the identified data object from a database. The retrieved content items can be used to generate a structured execution framework, which defines the arrangement, sequence, and/or transition logic for presenting therapeutic components within a digital therapeutic application.

The system can also process a treatment plan by modeling relationships between retrieved content items and therapeutic components within a structured execution framework. For example, retrieved content can include predefined therapeutic exercises, multimedia resources, and/or interactive interface elements. The structured execution framework organizes these components based on predefined timing parameters, user engagement rules, and/or conditional transitions derived from the directed process graph. The structured execution framework can further encode conditional logic governing when and how a treatment component is provided for presentation.

In some implementations, the system can generate a structured execution framework that incorporates predefined timing attributes, execution dependencies, and/or sequencing logic. The structured execution framework can define a hierarchical arrangement of treatment components. The treatment components can be arranged based on timing parameters specifying the sequence and duration of at least one of the plurality of treatment components. The structured execution framework can also define multiple execution paths corresponding to alternative treatment workflows.

Additionally, the systems and methods include generating structured treatment workflows that incorporate predefined conditional logic and structured relationships between treatment components. The structured execution framework can include a plurality of conditional logic associated with at least one treatment component and associates at least one subsequent treatment component with corresponding conditional logic. The subsequent treatment component can be linked to at least one prior treatment component based on predefined conditional logic corresponding to sequence constraints, and/or execution dependencies. For example, the structured execution framework can associate a breathing exercise with a subsequent treatment component based on predefined conditional logic rather than user-specific completion status. In another example, the structured execution framework can define transition conditional logic that specify when a treatment component is presented based on timing parameters or predefined relationships between treatment components. The structured execution framework can provide a structured representation of sequencing without requiring direct user input to determine treatment progression.

The system can also process structured treatment workflows by retrieving historical treatment component and configurations from a database. The system can query a database to identify at least one data object corresponding with a treatment component. The retrieved data object can include at least one content item and corresponding parameters. Corresponding parameters can include a content item relationship, a timing parameter, and/or metadata for structuring the retrieved action within the directed process graph. The retrieved content items can be incorporated into the structured execution framework to support digital therapeutic application modeling.

The systems and methods described herein improve upon traditional technical solutions by generating structured execution frameworks that facilitate efficient, scalable, and/or adaptable modeling of treatment workflows. By using generative AI models to curate treatment workflows, retrieve structured content, and/or define structured data representations, the disclosed system provides an improved technical solution for structuring digital therapeutic treatment plans. For example, the integration of directed process graphs, structured execution frameworks, and/or timing logic ensures that the generated treatment plans align with structured intervention models and can be integrated into digital therapeutic applications in a fast and scalable way that is also personalized to each user.

The systems and methods described herein can be used for a variety of purposes, including curating structured treatment plans, generating therapy workflows, modeling structured execution rules for therapeutic interventions, and/or integrating retrieved content into predefined treatment frameworks. Additionally, these methods can improve the efficiency of digital treatment planning by reducing reliance on manually defined treatment plan workflows, allowing structured modeling of therapeutic sequences, and/or providing technical consistency in treatment plan delivery.

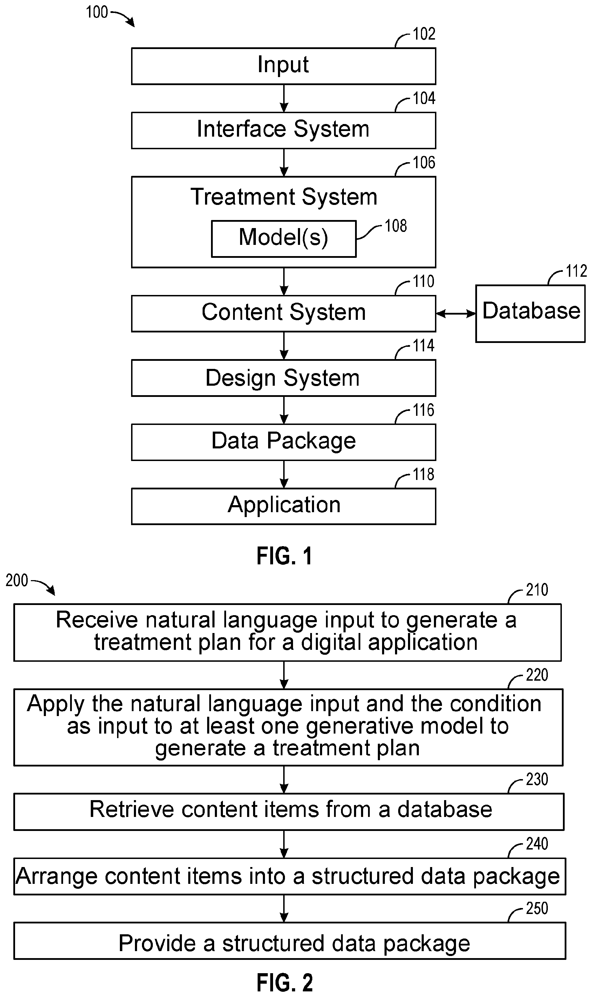

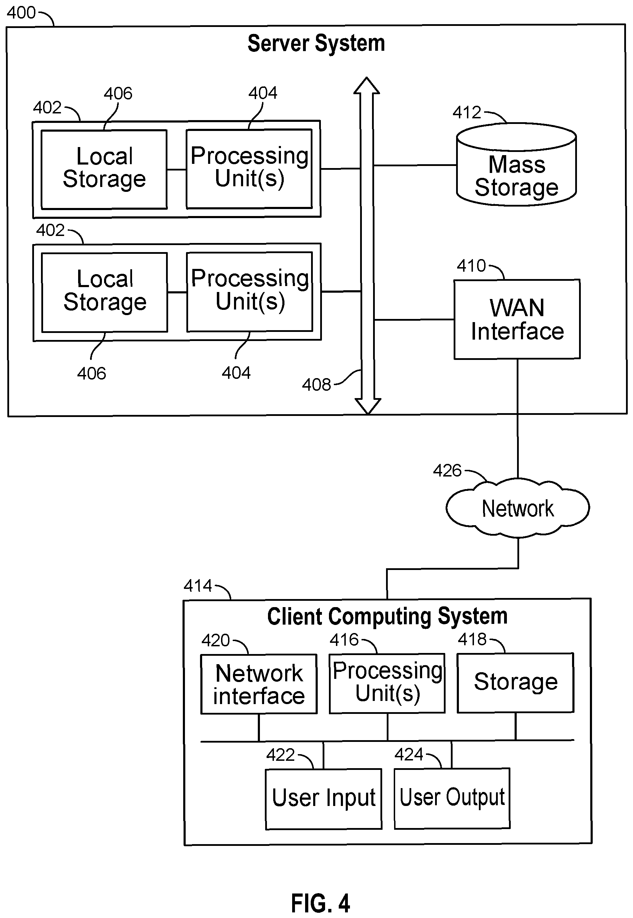

With reference to FIG. 1, FIG. 1 is a block diagram illustrating a system 100 in accordance with some implementations of the present disclosure. The system 100 includes components that can be implemented as discrete hardware, distributed components, and/or a combination of hardware, firmware, and/or software. The components of the system 100 can be configured to perform functions described herein, and/or the arrangement of these components can vary depending on the implementations. The arrangement shown in FIG. 1 is provided as an example and is not limiting. Other configurations and elements (e.g., machines, interfaces, functions, orders of operations, and/or groupings of functions) can be included in addition to and/or in place of those depicted. Some components can be omitted in particular implementations, depending on the operational requirements. Additionally, some functional entities in the system 100 can be grouped differently or implemented in alternative locations. The functions described in connection with the components of the system 100 can be performed by a processor and/or processors executing instructions stored in a memory. The instructions can direct the processor(s) to execute operations corresponding to one or more components of the system 100. The system 100 can also include interfaces or connections (e.g., wired and/or wireless communication technologies) that facilitate communication between components. The implementations can vary based on the requirements of the system and its operational environment. In some implementations, the systems, methods, and/or processes described herein can be executed using similar components, features, and/or functionality to those of example server system 400 of FIG. 4 and/or example administrator device 414 (depicted as "client computing system 414") of FIG. 4.

The system 100 can implement at least a portion of a digital therapeutic pipeline, such as a treatment planning pipeline, a content generation pipeline, a behavioral intervention pipeline, and/or a condition-specific therapeutic adaptation pipeline. The system 100 can generate structured execution frameworks to provide targeted therapeutic content for addressing various conditions. The system 100 can generate execution frameworks used to improve physical, cognitive, social, and/or behavioral skills and/or enhance such training by any of various systems described herein, including assertiveness training systems, emotion training systems, professional communication systems, adaptive language proficiency systems, social skills reinforcement systems, therapeutic intervention systems, role-based interaction platforms, and/or behavioral therapy support systems. Additionally, the system 100 can process natural language input using at least one generative model to create directed process graphs containing a plurality of actions and conditional logic. The system 100 can be used to generate adaptive digital treatment plans for mental health interventions, cognitive behavioral therapy modules, personalized rehabilitation programs, guided exposure therapy sequences, digital coaching systems, automated user progress tracking systems, and/or any adaptive therapeutic response frameworks for real-time and/or near real-time intervention adjustments.

Generally, the digital therapeutic pipeline can include operations performed by the system 100. The digital therapeutic pipeline can include any one or more of an interfacing stage, a modeling stage, content stage, framework stage and/or an outputting stage. Each stage of the digital therapeutic pipeline includes one or more components of the system 100 that perform the functions described herein. In some implementations, one or more of the stages can be performed during the training phase of AI models. Additionally, one or more of the stages can be performed during the inference phase using the AI models (e.g., model(s) 108).

The system 100 (e.g., implementing the digital therapeutic pipeline) can receive natural language input (e.g., input 102) to retrieve content from a database or generate content for a digital therapeutic application to address a condition and/or conditions (e.g., migraine). The system 100 implementing the digital therapeutic pipeline can apply the natural language input and the condition and/or conditions as the input 102 to at least one generative model to cause the at least one generative model to generate a directed process graph for the condition(s). The directed process graph includes a plurality of actions and conditional logic according to which to provide, for presentation, at least one of the plurality of actions. In some implementations, implementing the digital therapeutic pipeline can include the system 100 identifying (e.g., via an interface of a database) at least one data object (e.g., treatment component) corresponding with at least one of the plurality of actions included in the directed process graph.

Additionally, implementing the digital therapeutic pipeline can include the system 100 generating, using the at least one data object and the directed process graph, a structured execution framework. The generated structured execution framework can identify a plurality of content items identifying at least one of the plurality of actions and/or corresponding conditional logic according to which to provide, for presentation, at least one of the plurality of actions. In some implementations, implementing the digital therapeutic pipeline can include providing the structured execution framework. Thus, the digital therapeutic pipeline can provide technical improvements over prior therapeutic systems relying on predefined, static treatment plan generation, by applying generative models to construct structured execution frameworks for dynamic treatment plans.

In some implementations, the interfacing stage can be the stage in the digital therapeutic pipeline in which the system 100 can receive natural language input and facilitate interaction with a Large Language Model (LLM). The system 100 can include at least one interface system 104. The interface system 104 can receive natural language input, structured prompts, treatment-specific queries, user preferences, user interaction data, and/or any data relevant to treatment personalization or other user personalizations to retrieve content from a database or generate content for a digital therapeutic application to address a condition. The interface system 104 can receive natural language input (e.g., the input 102) to generate a treatment plan for a digital therapeutic application to address a condition. During the interfacing stage, the interface system 104 can capture, obtain, and/or format the free-text inputs to align with predefined LLM parameters (e.g., tokenization rules, response length constraints, semantic weighting, context preservation strategies, and/or any customized prompt engineering techniques).

In some implementations, the interface system 104 can receive and/or otherwise interface with an administrator and/or user device by establishing a secure communication channel for data exchange. The free-text can be queries, responses, comments, and/or contextual inputs provided by the administrator and/or user during the treatment plan generation process. The input 102 can be typed responses, speech-to-text conversions, structured feedback, session-specific phrases, gesture-based inputs, biometric sensor data, and/or any other form of data. In some implementations, the input 102 can also include recordings (e.g., audio/video), user annotations, context-aware prompts, sentiment data, timestamps, interaction history, physiological response metrics, and/or any additional data obtained before, during, or after a session. The interface system 104 can receive and/or otherwise obtain the input 102 from an administrator. The interface system 104 can receive and/or otherwise obtain the input 102 from a user device by polling the device during a treatment session or via a push notification system.

In some implementations, the interface system 104 can initiate a treatment plan generation process between the system 100 and an administrator providing natural language input. The natural language input can include instructions identifying treatment goals, treatment duration, therapeutic activities, actions corresponding to therapeutic activities, criteria for completing therapeutic activities, and/or other instructions used to generate a treatment plan for a digital therapeutic application (e.g., the application 118). The input 102 can be high-level treatment details (e.g., goals, duration, exercises, parameters for transitioning between exercises, and/or other details). The natural language input can be a prompt such as "create a 3-month treatment journey for an individual with PTSD who has triggers in the afternoon in the middle of the workday and on days in the middle of the workweek that includes a schedule for breathing exercises, grounding exercises, interactive therapeutic intervention activities, medication administration, and the first five days of treatment based on a predefined template." Additionally, the input can include additional instructions, such as "the first 5 days of treatment to be based on our treatment template where if the user answers yes, the treatment will transition on the next screen." In some implementations, an administrator can provide the input 102. An administrator can include an operator, developer, clinician, researcher, healthcare provider, regulatory specialist, pharmaceutical individuals, and/or system reviewer with authorized access to generate and/or refine treatment workflows and/or adjust content presentation in the digital therapeutic system.

Additionally, when multiple data points and/or elements (e.g., multiple contexts, treatment journey having session objectives, performance history, and/or ongoing metrics) are received by the interface system 104, the interface system 104 can prioritize based on predefined weights and/or hierarchical rules to process the input. The interface system 104 can resolve conflicting inputs and/or ambiguous data using a decision-making algorithm and/or function (e.g., machine learning models trained on historical data, predefined priority rules, user-specific preferences, heuristic-based ranking methods, and/or any context-aware optimization techniques).

Additionally, the digital therapeutic application (e.g., the application 118) can be configured to provide input for the interface system 104. The application 118 can provide an intuitive and engaging interface for an administrator and/or user to interact with the system 100 to determine and/or generate personalized therapeutic goals, treatment journey, user preferences, cognitive capacity, and/or any relevant treatment plans. In some implementations, application users can interact with the system 100 via a digital therapeutic application to construct a digital therapeutic journey.

A user device can be operating and/or otherwise implementing a digital therapeutic application (e.g., mobile app, web-based platform, desktop software, and/or any compatible user interface). In some implementations, the user device can be a smartphone, tablet, laptop, desktop computer, wearable device, and/or any internet-configured device. The user device can serve as the medium for providing the data for the treatment plan generation system. The user device can allow real-time and/or near real-time data exchange between the user and the interface system 104.

Natural language input can include input from pre-generated treatment plans, including treatment plans defined in a digital therapeutic application (e.g., the application 118) that can communicate with the interface system 104 to transmit natural input. The interface system 104 can transmit a request to interact with predefined data (e.g., context, session history, user preferences, and/or any behavioral patterns) or contextual information about a treatment plan or session with an end user addressing a condition and/or conditions (e.g., nausea) via a digital therapeutic application.

In some implementations, the modeling stage can be the stage in the digital therapeutic pipeline in which the system 100 can apply inputs to generate a treatment plan (e.g., directed process graph) for a digital therapeutic application. The system 100 can include at least one treatment system 106. The treatment system 106 can apply the input (e.g., natural language input) to at least one generative model (e.g., the model(s) 108) to generate a directed process graph for the content system 110. The directed process graph can include a plurality of actions and conditional logic according to which to provide, for presentation, at least one of the plurality of actions. The treatment system 106 can process natural language inputs to cause the at least one generative model to generate a treatment plan. The treatment plan can include a list of treatment components and a schedule for presenting at least one treatment component of the list of treatment components that align with the therapeutic goals of the input.

The at least one generative model can be a deep neural network, language model, large language model (LLM), small language model (SLM), vision language model (VLM), multimodal language models (MMLM), perception models, tracking models, fusion models, transformer models, diffusion models, encoder-only models, decoder-only models, encoder-decoder models, neural rendering field (NERF) models, diarization models, and/or transcription models, among others to generate the directed process graph. Causing the model(s) 108 to generate the directed process graph can include processing the natural language input to extract a plurality of actionable elements, associating the plurality of actionable elements with the plurality of actions, and/or arranging the plurality of actions into a hierarchical structure. The model(s) 108 can be a language model that can process natural language input to generate a smoking cessation treatment plan for nicotine dependence, a migraine treatment plan for episodic and chronic migraine, a multiple sclerosis treatment plan for symptom management and relapse prevention, an atopic dermatitis treatment plan for flare-up control and long-term skin barrier maintenance, an obesity treatment plan for lifestyle intervention and pharmacotherapy guidance, an oncology treatment plan for symptom management and psychological support, an insomnia treatment plan for sleep hygiene reinforcement and behavioral therapy, an acute coronary syndrome treatment plan for post-event rehabilitation and secondary prevention, and/or any condition(s)-specific therapeutic treatment plan. The model(s) 108 can extract a plurality of actionable elements, such as "identify negative thought patterns," "introduce cognitive restructuring exercises," "perform breathing exercises," "use grounding techniques," "schedule mood tracking," "provide relapse prevention strategies," "track medication adherence," "assess behavioral triggers," "implement progressive exposure therapy," "schedule automated check-ins," and/or any structured therapeutic guidance components.

The model(s) 108 can associate these actionable elements with a plurality of actions, such as presenting psychoeducational lessons, prompting user reflections, and/or providing interactive exercises, delivering personalized recommendations, generating progress reports, dynamically adjusting treatment pathways, and/or any adaptive therapeutic intervention while linking them to a plurality of conditional logic, such as completion of prior therapeutic activities, user-reported symptom changes, biometric feedback (e.g., heart rate variability), engagement duration, a particular day or time, user preferences, and/or other conditional logic. The model(s) 108 can further arrange the plurality of actions and the plurality of conditional logic into a hierarchical structure, organizing the directed process graph to introduce psychoeducation, progress to interactive cognitive restructuring exercises, and/or adjust follow-up interventions based on user engagement and reported progress.

In some implementations, the plurality of actions can correspond to a treatment component, a session, a prompt, a task, a response, and/or any interactive or passive therapeutic engagement method. The design system 114 can generate a data object corresponding to a diagnostic query for providing a treatment for smoking cessation, migraines, multiple sclerosis, atopic dermatitis, obesity, oncology, insomnia, acute coronary syndrome, and/or any health condition.

The system 100 can structure a data object to include a diagnostic screening process or clinical scale or questionnaire containing a sequence of standardized questions, predefined scoring logic, and/or conditional branching rules. The system 100 can generate a data object to determine symptom severity, determine whether further evaluation is necessary, and/or dynamically adjust the progress graph by incorporating targeted interventions, such as behavioral activation exercises or guided cognitive restructuring sessions. The data object can include logic for responding to a completion of the treatment component or providing the response to the diagnostic or clinical scale/questionnaire query, updating an activity record to include a completion status, timestamp data, and/or data generated or obtained during the treatment component or diagnostic or clinical scale/questionnaire query. A data object can include logic connecting logging the completion status, recording the duration of engagement, capturing biometric data (e.g., heart rate variability from a wearable device), and/or updating the content based on this information.

In some implementations, the treatment system 106 can maintain, execute, train, and/or update one or more machine-learning models during the encoding stage. In some implementations, the machine-learning model(s) can include any type of generative and predictive machine-learning models capable of processing natural language input to generate structured therapeutic workflows (e.g., deep learning models, transformer-based models, and/or sequence-to-sequence models) to produce directed process graphs representing treatment plans.

The machine-learning model(s) can be trained and/or updated to extract actionable elements from natural language descriptions of treatment plans, associate these elements with predefined therapeutic components, and/or generate structured execution frameworks, among other adaptive treatment plan generation tasks. The machine-learning model(s) can be or include a transformer-based model (e.g., a generative pre-trained transformer (GPT) model). The machine-learning model(s) can be or include a reinforcement learning-based model, in some implementations.

The model(s) 108 can include an encoder configured to execute the machine-learning model to generate outputs. The encoder can receive data to provide as input to the machine-learning model(s), which can include natural language input (e.g., from an operator). The encoder can process operator-provided treatment goals and parameters, extract structured actions and conditional logic, and/or generate a directed process graph representing a sequence of therapeutic components corresponding to conditional logic.

Additionally, the model(s) 108 can be a supervised learning model that can process labeled treatment session transcripts to extract a plurality of actionable elements for a program. The model(s) 108 can be trained on annotated datasets containing structured user-provider interactions to identify key therapeutic components, such as "introduce distress tolerance techniques," "reinforce mindfulness exercises," and "assess emotional regulation progress." The model(s) 108 can associate these actionable elements with a plurality of actions, such as delivering guided meditation, prompting self-assessment quizzes, generating personalized feedback, adjusting treatment recommendations, modifying session pacing, presenting interactive educational content, suggesting strategies, offering real-time adaptive interventions, modifying the difficulty level, and/or any action facilitating therapeutic engagement and/or disengagement while linking them to a plurality of metrics, such as user engagement scores, completion of assigned exercises, and/or predefined behavioral milestones. The model(s) 108 can further arrange the plurality of actions and the plurality of conditional logic into a hierarchical structure, such as by structuring the directed process graph to introduce mindfulness techniques, transition to distress tolerance exercises based on engagement levels, and/or personalize emotional regulation strategies depending on user performance.

As used herein, "directed process graph" can refer to a structured representation of actions, conditional logic, and transitions within a treatment workflow, defining relationships between therapeutic steps and their dependencies. The directed process graph represents the sequence in which therapeutic activities, treatment plans, and/or user interactions are executed, including any branching logic based on engagement levels or predefined criteria. The directed process graph can specify that a user completes an initial module before progressing to treatment component, with conditional transitions based on user responses or assessment results. Thus, it should be understood that the directed process graph structures therapeutic workflows to dynamically adapt based on user inputs, predefined logic, and/or evolving treatment requirements.

Additionally, the model(s) 108 can be an unsupervised learning model that can analyze unstructured user journaling data to extract a plurality of actionable elements for a digital therapeutic treatment plan targeting a condition. The model(s) 108 can cluster user-generated text entries based on recurring themes, such as "heightened stress levels," "negative self-talk," and "avoidance behaviors," without requiring predefined labels. The model(s) 108 can associate these actionable elements with a plurality of treatment components, such as suggesting relaxation exercises, providing cognitive reframing techniques, and/or guiding users through exposure tasks, while linking them to a plurality of conditional logic, such as detected sentiment polarity, frequency of stress-related keywords, and/or patterns of user engagement. The model(s) 108 can further arrange the plurality of treatment components and the plurality of conditional logic into a hierarchical structure, generating a directed process graph that includes conditional logic that can be used to dynamically adapt the therapeutic flow. The model(s) 108 can arrange the plurality of treatment components and plurality of conditional logic by recognizing emerging anxiety patterns, personalizing therapeutic exercises, and/or adjusting treatment pathways in real time based on evolving user inputs.

The directed process graph can correspond to a representation of user interaction flow (e.g., treatment plan), including therapeutic components and corresponding transitions, structured to define the treatment journey over a time period (e.g., daily sessions, weekly progress evaluations, adaptive treatment plan checkpoints, phase-based treatment milestones, dynamically adjusted timelines, and/or any structured temporal framework). The directed process graph can include a graph with nodes and edges for a treatment plan for various conditions. The nodes can represent individual therapeutic activities, assessment checkpoints, educational modules, user decision points, automated feedback triggers, and/or any treatment component. The edges can represent conditional transitions between activities, branching logic for adaptive treatment pathways, dependencies based on user progress, reinforcement loops for skill consolidation, and/or any structured relationship governing progression. The interaction flow over time can guide the user through progressive therapeutic steps (e.g., nodes). The directed process graph can include an initial assessment phase pointing to components of the treatment plan (e.g., structured exposure exercises, coping strategy reinforcement, and/or periodic self-assessment check-ins, and/or other modules). At least one (e.g., each) stage can include specific actions, such as guided breathing exercises, journaling prompts, virtual coaching sessions, mindfulness challenges, symptom tracking entries, role-playing simulations, psychoeducational lessons, interactive scenario-based training, and/or any structured treatment component with conditional logic (e.g., schedule, completion criteria, user-reported outcomes, adaptive difficulty adjustments, real-time and/or near real-time performance monitoring, automatic intervention escalation, personalized content recommendations, and/or any dynamic treatment adaptation logic) determining progression based on user responses and engagement levels, among other examples.

In some implementations, a treatment plan can include the schedule identifying a plurality of conditional steps corresponding with at least one of the list of treatment components of a treatment journey. The directed process graph can include a hierarchical arrangement of components representing the plurality of actions, the model(s) 108 generating the hierarchical arrangement arranged based on the plurality of timing parameters identifying a sequence and duration corresponding with at least one of the plurality of actions. Additionally, the plurality of conditional steps can correspond with conditional logic to satisfy. The model(s) 108 can generate a treatment plan for post-traumatic stress disorder (PTSD) that includes a schedule with conditional steps guiding the user through exposure therapy. The model(s) 108 can structure the treatment journey to include an initial education phase, followed by gradual exposure exercises. The treatment journey can include a node containing data regarding the initial education phase and edges pointing to gradual exposure exercises to address the initial phase. The plurality of conditional steps can correspond with conditional logic such as user-reported anxiety levels, completion of assigned exercises, and/or engagement with coping strategies. If the user successfully completes a guided breathing session and reports reduced anxiety, the model(s) 108 can progress to the next exposure task. Otherwise, the system can adjust the difficulty or provide alternative coping mechanisms before advancing.

In some implementations, the schedule of the treatment plan can include a plurality of conditional logic corresponding with at least one treatment component and initiating at least one subsequent treatment component. The at least one subsequent treatment component can be provided based on a completion of or failure of at least one prior treatment component. The graph can provide logic for if a user successfully completes a mindfulness meditation session (e.g., maintains focus for a designated duration) where the scheduling logic for the plan can indicate to schedule a more advanced mindfulness practice as the subsequent treatment component. The graph can include a node for a treatment component, with an edge pointing to future activities if the user completes the at least one prior treatment component and an edge pointing to different future activities if the user fails to complete the at least one prior treatment component.

The graph can provide conditional logic for if a user successfully completes a migraine session (e.g., reports reduced migraine frequency and severity over a predefined period) where the conditional logic for the treatment plan can indicate to progress to advanced pain management strategies and/or trigger a follow-up assessment to monitor long-term effectiveness. The graph can include node(s) for migraine sessions, with edges pointing to actions for migraine management.

The graph can provide conditional logic for if a user successfully completes a smoking cessation session (e.g., maintains nicotine abstinence for a predefined duration) where the conditional logic for the treatment plan can indicate to reinforce relapse prevention techniques and/or introduce long-term behavior maintenance strategies. The graph can include node(s) for smoking cessation sessions, with edges pointing to actions for nicotine treatment.

The graph can provide conditional logic for if a user successfully completes an obesity treatment session (e.g., achieves a target level of dietary adherence and physical activity engagement) where the conditional logic for the treatment plan can indicate to initiate metabolic health monitoring and/or introduce new goal-setting modules for sustained weight management. The graph can include node(s) for obesity treatment sessions, with edges pointing to actions for physical activities.

The graph can provide conditional logic for if a user successfully completes an oncology treatment session (e.g., demonstrates adherence to symptom management techniques and reports improved quality of life metrics) where the conditional logic for the treatment plan can indicate to transition to survivorship care planning and/or incorporate ongoing psychosocial support. The graph can include node(s) for oncology treatment sessions, with edges pointing to actions for symptom management.

In some implementations, if the user fails to complete the session (e.g., reports significant difficulty maintaining focus) and/or fails to engage with assigned therapeutic exercises for a predefined period, the knowledge graph can include conditional logic to repeat the session with additional guidance or support tools, such as a shorter duration or added instructional prompts. Additionally, the at least one subsequent treatment component can be provided based on a completion of, partial completion of, and/or a failure to meet predefined criteria for the at least one treatment component. For example, if a user fails to meet the predefined criteria of journaling daily reflections (e.g., misses two consecutive entries), the knowledge graph can include conditional logic for introducing a simplified journaling prompt or an alternative reflective exercise to re-engage the user. If the user meets the criteria, the knowledge graph can point the treatment plan to a node for integrating these reflections into a cognitive restructuring exercise, advancing the therapeutic journey. Nodes of the directed process graph can include edges corresponding to transitions between therapeutic activities based on completion status, partial completion, and/or failure to meet predefined criteria. Each edge can represent a conditional pathway where the next node in the treatment sequence is determined by real-time user engagement data and/or healthcare provider (HCP) recommendation. As another example, if a user successfully completes a mindfulness session, an edge from the current node can point to a more advanced relaxation technique module. If the user disengages or fails to meet predefined thresholds (e.g., exits early or reports persistent stress), an alternative edge can redirect the process graph to a remedial technique, such as an instructional video or guided support session. The edges of the directed process graph can incorporate weighted probabilities or reinforcement learning mechanisms to refine transitions over time.

As used herein, "conditional logic" can refer to rules governing the transition between therapeutic components based on user responses (including a lack of responses), system-detected behavioral patterns, and/or other predefined criteria (e.g., a passage of time). The conditional logic can determine whether a user advances, repeats, and/or modifies a treatment component based on engagement levels, assessment scores, and/or other predefined thresholds. For example, if a user completes a breathing exercise with low engagement, the system can schedule an alternative relaxation technique before progressing to the next phase. Thus, it should be understood that conditional logic facilitates personalized treatment pathways (e.g., edges) by dynamically adjusting therapeutic steps based on real-time and/or near real-time user interaction data and/or HCP recommendation.

As used herein, a "component" of a treatment plan can refer to a module, activity, intervention, and/or group thereof. A component can consist of one or more therapeutic activities designed to address a specific aspect of treatment (e.g., symptom management, skill-building, behavioral modification, and/or other aspects). A component of the treatment plan can include sessions, activities, interventions, and/or other therapeutic steps for a treatment plan. Thus, it should be understood that a component represents one or more individual activities of a treatment plan workflow, structured to support progression through the overall treatment journey.

As used herein, a "session" can refer to a discrete unit of therapeutic interaction within a treatment plan, structured to deliver targeted interventions, assessments, and/or educational content. A session can consist of one or more therapeutic activities designed to be completed within a specific timeframe, often accompanied by progress tracking and engagement metrics. For example, a session can include a guided mindfulness exercise, a cognitive restructuring task, and/or a mood-tracking questionnaire to be completed in a single sitting. Thus, it should be understood that a session represents an individual activity of a structured treatment plan or component of a structured treatment plan, facilitating progress toward a treatment goal.

As used herein, an "action" can refer to a specific therapeutic activity and/or system-driven process executed within a session to advance treatment goals. An action can correspond to an operational step in the treatment workflow, such as presenting an educational module, triggering a behavioral prompt, and/or collecting user-reported data. The action can involve displaying a psychoeducational video, initiating a guided breathing exercise, and/or dynamically adjusting content based on user engagement. Thus, it should be understood that an action serves as a functional component within a directed process graph, facilitating structured intervention delivery and real-time and/or near real-time adaptability.

As used herein, a "condition" can refer to a disease, disorder, symptom, or other condition of a user. A condition can correspond to the targeted underlying health condition of a user the treatment plan is designed to address.

In some implementations, directed process graph can include a plurality of execution paths. The plurality of execution paths of the process graph can correspond to a plurality of treatment workflows of a treatment journey. As an example of a digital therapeutic program addressing insomnia, the plurality of execution paths can correspond to distinct treatment workflows, such as a sleep hygiene education workflow, a cognitive restructuring workflow for negative sleep thoughts, and/or a stimulus control therapy workflow. At least one (e.g., each) execution path can be dynamically selected (e.g., by the model(s) 108) based on user-reported sleep patterns and engagement with prior interventions. Additionally, at least one (e.g., each) treatment workflow of the plurality of treatment workflows can include a sequence of therapeutic components to address or manage the condition. The cognitive restructuring workflow for insomnia can include an initial sleep diary assessment, followed by guided exercises to challenge negative beliefs about sleep, personalized relaxation techniques, and/or scheduled sleep restriction interventions. Progression through these actions can be determined by user-reported improvements in sleep efficiency and adherence to recommended interventions.

In some implementations, the content stage can be the stage in the digital therapeutic pipeline in which the system 100 can identify at least one data object corresponding with at least one of the plurality of actions included in the directed process graph. The system 100 can include at least one content system 110. The content system 110 can interface with a database 112 to apply actions included in the directed process graph. The content system 110 can retrieve, via an interface of the database 112, from a database a plurality of content items corresponding with at least one treatment component of the list of treatment components in the treatment plan.

The content system 110 can retrieve, for example, a breathing exercise module, a progressive muscle relaxation submodule, behavior cessation module, migraine module, multiple sclerosis module, atopic dermatitis module, obesity module, oncology module, insomnia module, acute coronary syndrome module, and/or any therapeutic treatment plan module corresponding to a structured treatment plan. Additionally, the content system 110 can retrieve and/or otherwise obtain sub-modules of various modules by accessing predefined content hierarchies, querying a database (e.g., database 112) for related therapeutic components, dynamically generating sub-modules based on treatment parameters, referencing prior user engagement data, and/or any adaptive content retrieval mechanism supporting modular treatment customization. In some implementations, various templates can be obtained via an interface of the database 112. The content system 110 can use the directed process graph to identify and retrieve relevant therapeutic content items for use by the design system 114 to generate a structured execution framework.

The database 112 can be internal or external to the system 100. In some implementations, the database 112 can correspond to an external repository (e.g., Figma, Adobe XD, GitHub, and/or any other repository) accessed via an interface (e.g., application program interface (API), webhooks, SDK, GraphQL query, and/or any other interface) to retrieve existing modules, submodules, and/or configuration data for incorporation into the digital therapeutic application. The content system 110 can interface with a digital experience platform (e.g., Adobe XD, Sketch, Figma, InVision, Zeplin, and/or other platforms) to retrieve a structured template for a treatment plan, including predefined UI components for psychoeducation, interactive exercises, and/or progress tracking. The content system 110 can query an API to extract design tokens and layout structures. In some implementations, database 112 can correspond to an internal repository contained within system 100 for storing preconfigured treatment workflows, reusable therapeutic modules, and/or adaptive conditional logic rules within the system 100 (e.g., application 118). The content system 110 can access the internal repository to retrieve and modify structured execution frameworks without relying on external design tools.

In some implementations, identifying at least one data object can include querying the database 112 maintaining the at least one data object, and/or querying the database 112 can include obtaining, using at least one API request, the at least one data object corresponding to at least one therapeutic action or configuration. The content system 110 can query the database 112 to retrieve a previously configured treatment plan. The content system 110 can send an API request to the database 112 to obtain a data object corresponding to a therapeutic action for the user to perform, such as a structured exposure therapy session. The retrieved data object can include session structure, pacing guidelines, user interface components for rendering therapeutic content, interactive elements for user engagement, transition logic for navigating between treatment steps, UI state parameters for dynamic content adaptation, accessibility configuration, and/or conditional branching logic based on user progress and/or HCP recommendations.

In some implementations, the at least one data object includes at least one of the plurality of content items and corresponding parameters. A data object can be a content item, including corresponding parameters such as duration, difficulty level, and/or guided instruction text. The content system 110 can retrieve this data object and adjust its parameters based on a treatment phase or preferences of the user or other examples. Additionally, the corresponding parameters can include at least one of a content item relationship, a timing parameter, metadata for representing the at least one therapeutic action as a node or relationship in the directed process graph. A therapeutic action, such as a graded exposure module for treatment, can be represented in the directed process graph as a node connected to multiple branching paths. These paths can correspond to different exposure levels, where progression depends on user-reported anxiety reduction, as an example. The system can use metadata, such as prior user completion rates and effectiveness scores, to determine optimal exposure levels dynamically. The content can be personalized to user preferences, such as a user indicating (or the system detecting) that a summer scene is more calming to the user than a winter scene, or that a city landscape is more engaging than a nature landscape, or that avatars in a different demographic group than the user is more encouraging when presenting psychoeducation lessons.

In some implementations, the framework stage can be the stage in the digital therapeutic pipeline in which the system 100 can generate a structured execution framework. The system 100 can include at least one design system 114. The design system 114 can include any one or more artificial intelligence models (e.g., machine learning models, supervised models, language models, LLMs, SLMs, VLMs, MMLMs, neural network models, deep neural network models), rules, heuristics, algorithms, functions, and/or various combinations thereof to perform operations including receiving, applying, identifying, causing, generating, administering, and/or providing a structured execution framework. The design system 114 can include a neural network to generate, using at least one data object and the directed process graph, a structured execution framework.

The design system 114 can generate the structured execution framework to organize and/or otherwise arrange a plurality of content items identifying at least one of the plurality of actions. Additionally, the design system 114 can generate a structured execution framework to arrange corresponding conditional logic according to which to provide, for presentation, at least one of the plurality of treatment components. The design system 114 can arrange, using the plurality of content items and/or the schedule, the list of treatment components of the treatment plan into a structured data package for presentation in the digital therapeutic application.

In some implementations, the design system 114 can maintain, execute, train, and/or update one or more machine-learning models. In some implementations, the machine-learning model(s) can include any type of deep learning-based, sequence-to-sequence, and/or rule-based machine-learning models capable of generating a structured execution framework (e.g., pseudocode, skeleton code, workflow graphs, execution state diagrams, hierarchical action mappings, declarative configuration files, API interaction schemas, and/or any structured representation facilitating treatment plan execution) to construct an adaptive digital therapeutic application based on natural language treatment goals and parameters. The design system 114 can implement one or more machine-learning models to improve conditional logic for treatment progression, and/or generate executable workflow representations, among other tasks such as refining content personalization, adjusting treatment workflows based on user engagement, and/or predicting optimal intervention sequences to arrange a structured execution framework.

As used herein, a "structured execution framework" and/or "structured data package" can refer to a representation of organized data, relationships, and/or execution parameters that define how a treatment plan is structured, processed, and implemented within a digital therapeutic application. The structured execution framework provides a formalized structure for encoding actions, conditions, and/or dependencies, allowing processing, execution sequencing, and/or integration with external systems (e.g., application 118). The structured execution framework can specify a sequence of therapeutic activities, corresponding transitions, timing constraints, and/or conditional logic for adapting treatment delivery. Thus, it should be understood that the structured execution framework supports dynamic and structured execution of treatment workflows, facilitating modularity, adaptability, and/or integration across various digital therapeutic applications.

The machine-learning model(s) can be or include a transformer-based model (e.g., a generative pre-trained transformer (GPT) model). The machine-learning model(s) can be or include a reinforcement learning-based model, in some implementations. Design system 114 can execute the machine-learning model to generate outputs. The design system 114 can receive data to provide as input to the machine-learning model(s). In some implementations, the system 100 can configure (e.g., train, update, fine tune, apply transfer learning to) the model(s) of the design system 114 by modifying or updating one or more parameters, such as weights and/or biases, of various nodes of the model(s) responsive to evaluating estimated outputs of the model(s).

During the framework stage, the design system 114 can process a natural language-defined treatment plan and generate a structured execution framework by extracting treatment components from a database. The design system 114 can use the directed process graph to associate exercises, activities, and/or tasks with corresponding conditional logic, such as user engagement metrics and predefined success criteria.

In some implementations, the structured execution framework, and/or structured data package, corresponds to a representation of the treatment plan generated from the directed process graph and/or the data objects. The structured execution framework can correspond to the plurality of treatment components, conditional logic, and/or timing attributes organized to support generating executable instructions for constructing a digital therapeutic application. The design system 114 can convert a structured execution framework into a data package by encoding the plurality of actions, conditional logic, state transitions, user interaction parameters, execution dependencies, and/or timing attributes into a JSON, XML format, YAML, protocol buffers (protobuf), and/or any configuration file. The structured data package can serve as an intermediary representation for a digital therapeutic application to interpret and render the treatment sessions. The JSON file can define a sequence of treatment components, conditional logic for transitioning between activities, and/or timing constraints, allowing the system to dynamically generate user-facing interfaces. A configuration file can define UI layouts, specify conditional logic for rendering dynamic content updates, establish rules for adaptive content presentation, encode accessibility settings, and/or facilitate integration with external data sources or APIs.

The design system 114 can generate the structured execution framework by processing the directed process graph in conjunction with the obtained data objects to define executable treatment workflows. The design system 114 can traverse the directed process graph to identify nodes representing therapeutic components, extract associated conditional logic, and link these elements with relevant data objects. The design system 114 can arrange the actions, logic, elements, and/or other node objects into a structured data format (e.g., JSON, XML, YAML, and/or other data formats). For example, if the directed process graph includes a user assessment node followed by a relaxation module, the design system 114 can retrieve predefined content templates (e.g., a guided module from a database) and encode its execution parameters-such as duration, user progression criteria, and transition conditional logic-into the structured execution framework.

The design system 114 can use a hierarchical structuring approach to organize the execution framework based on relationships between directed process graph components and corresponding data objects. The design system 114 can analyze execution dependencies and conditional logic within the directed process graph to determine sequencing for treatment workflows. The design system 114 can then assign timing parameters, state transitions, and adaptive logic rules, generating an execution model that supports adjustments. For example, if a treatment journey includes progressive exposure therapy tasks, the design system 114 can retrieve intensity-adjusted task parameters from a structured data object, encode progression logic into an execution framework, and specify UI configurations that dynamically update based on user performance.

In some implementations, the structured execution framework, and/or structured data package, corresponds to ready-to-use format and/or scheme (e.g., machine-readable, API-compatible, platform-independent, extensible) structured for rendering the treatment journey that defines how at least one (e.g., each) content item is presented and specifies the sequence and timing of the content items. The structured execution framework can be formatted as a standardized schema, such as a state machine model, defining how content items are presented over time. The structured execution framework can specify whether a treatment component is presented before or after another treatment component based on real-time and/or near real-time user performance, administrator controls, and/or HCP recommendations. The design system 114 can generate this format as a structured object containing execution rules, metadata, and/or user progression paths.

In some implementations, the design system 114 can generate, using the structured execution framework, a plurality of instructions configured to cause the digital therapeutic application to present the content including at least one of the plurality of content items according to the directed process graph. The design system 114 can generate executable instructions for the digital therapeutic application, such as transforming the structured execution framework into an API-driven workflow that dynamically loads and presents therapeutic content. The design system 114 can take a treatment plan, generate corresponding JavaScript and Python instructions, and/or provide an interactive user experience through an adaptive digital interface. The system can compile the structured execution framework into executable functions that trigger content modules, dynamically adjusting them based on user progress, administrator controls, and/or HCP recommendations.

In some implementations, the structured execution framework corresponds to a mapping of the plurality of content items, the corresponding conditional logic (e.g., schedule), a plurality of timing parameters, and/or a plurality of execution dependencies of the directed process graph. As an example for a smoking cessation treatment plan, the design system 114 can generate a structured execution framework that includes a mapping of content items (e.g., motivational coaching videos, craving management exercises, behavioral substitution prompts), corresponding conditional logic (e.g., progress-dependent branching pathways based on reported cravings), timing parameters (e.g., daily self-assessment check-ins, weekly behavioral goal-setting sessions), and/or execution dependencies (e.g., prerequisite completion of nicotine replacement therapy education before introducing behavioral coping strategies) for a coherent and personalized treatment workflow in the digital therapeutic application.

In some implementations, the design system 114 can emulate, using an emulator, performance of the plurality of actions based on processing at least a portion of the plurality of instructions according to the structured execution framework. The plurality of instructions can include at least one operation for performing at least one of the plurality of actions corresponding with at least one timing parameter or conditional logic parameter. The design system 114 can use a reinforcement learning-based model and an emulator to simulate the execution of a PTSD treatment journey, as an example. The system 100 can process the structured execution framework in a sandboxed environment, predicting how users would interact with the therapeutic content. The emulator can analyze engagement trends, test different conditional pathways, and/or refine the execution framework before deploying it in the live digital therapeutic application.

In some implementations, the design system 114 can retrieve from a database or generate, using the structured execution framework and/or the directed process graph, skeleton code including at least one programmatic construct corresponding with the plurality of actions and corresponding conditional logic. The design system 114 can retrieve from a database or generate skeleton code from the directed process graph by extracting a sequence of therapeutic actions for the user to perform, mapping them to predefined functional treatment components, and structuring them into executable programmatic constructs. The design system 114 can analyze nodes and edges of the directed process graph, identifying dependencies between treatment components, state transitions, and conditional logic, and then translate this information into a structured code representation. If the directed process graph specifies a therapy sequence with conditional branching based on user-reported data, as an example, the design system 114 can generate JavaScript functions that define interactive session logic, such as event handlers for user responses, API calls for progress tracking, and UI state updates that adapt the treatment workflow dynamically.

Additionally, the design system 114 can compile skeleton code retrieved from at least one database. The design system 114 can retrieve skeleton code from a backend database containing predefined UI components for therapy modules. The system can structure the skeleton code to align with the directed process graph, generating a foundational codebase for a digital therapeutic application (e.g., application 118). The design system 114 can retrieve a template-based UI structure for a module from a database, extract relevant functional components (e.g., journaling input fields, guided breathing exercise templates), and integrate with logic derived from the directed process graph. The system can compile the retrieved skeleton code with dynamically generated logic from the structured execution framework. If the directed process graph specifies conditional logic for transitioning between therapy modules, the design system 114 can embed corresponding state management logic within the skeleton code.

In some implementations, using the structured execution framework, the system 114 can generate HTML templates defining the sequence of treatment sessions and interactive prompts. Using the directed process graph, the design system 114 can dynamically structure JavaScript functions that adapt content delivery based on user behavior, administrator controls, and/or HCP recommendations. Additionally, the design system 114 can retrieve from a database or generate skeleton code including JavaScript code for handling user interactions, managing state transitions, rendering treatment components dynamically, integrating API calls for progress tracking, and/or other JavaScript implementations in a digital therapeutic application or CSS code for styling treatment session UI elements, providing responsive design across devices, customizing animations for interactive treatment components, defining accessibility features for enhanced usability, and/or other implementations for use in development of or updates to a digital therapeutic application.

In some implementations, the design system 114 can provide the structured data package (e.g., content, treatment plan), and/or the skeleton code to an administrator for review. In some implementations, the design system 114 can receive, from an administrator, at least one update to the treatment plan or the skeleton code prior to providing the structured execution framework to the digital therapeutic application (e.g., application 118). For example, an administrator reviewing an AI-generated exposure therapy sequence for phobia treatment can adjust the intensity levels of initial exposure tasks before the design system 114 finalizes the structured execution framework for deployment in the digital therapeutic application. In some implementations, the design system 114 can receive, from an administrator, at least one update to the treatment plan or the skeleton code prior to using the skeleton code in the digital therapeutic application. A system engineer can update the skeleton code to integrate an additional progress-tracking API before deploying the code to the digital therapeutic application.

In some implementations, the outputting stage can be the stage in the digital therapeutic pipeline in which the system 100 can provide the structured execution framework. The system 100 can include at least one data package 116. In some implementations, the data package 116 can be the structured execution framework, structured data package, skeleton code, compiled workflow instructions, UI component mappings, and/or any other data package/object the design system 114 can provide discussed herein (e.g., to the application 118). The design system 114 can generate and send outputs to guide the application 118. The design system 114 can provide the data package 116 to a digital therapeutic application, which parses the structured execution framework to dynamically construct executable code to provide an interactive module. The application 118 can use the package to configure session flow, adjust content presentation based on conditional logic, and/or generate UI components for treatment components. The application 118 can process the structured data package to determine the sequencing of treatment components, adapt interactive elements based on predefined conditional logic, and/or organize content presentation according to treatment parameters. For example, if the data package specifies a progressive relaxation program, the application 118 can dynamically structure the session by generating an introductory breathing exercise, followed by a guided body scan, and adjusting subsequent activities based on user engagement levels.

The application 118 can be installed and/or otherwise maintained by a user device (e.g., mobile phone, tablet, wearable device, desktop computer, smart speaker, and/or any compatible hardware). The application 118 can receive the data package 116 for parsing structured execution frameworks, extracting treatment plan parameters, and configuring application behavior accordingly. The application 118 can interpret the structured data package in a runtime environment to organize and present therapeutic workflows based on system-generated execution dependencies. The data package 116 can include JSON-encoded treatment structure definitions specifying a multi-step migraine reduction program, as an example. Upon receiving this package, the application 118 can assemble a structured sequence of treatment components, adjusting UI elements and interactive components according to the encoded treatment plan. The data package 116 can include media references and instructional prompts for guided relaxation sessions. The application 118 can dynamically retrieve and present the corresponding audio, video, and/or textual content, structuring the user experience based on predefined scheduling parameters rather than executing predefined application logic.

Generally, the system 100 addresses the technical problems of providing effective and dynamic solutions for a large number of users by facilitating adaptive treatment workflows that personalize digital therapeutic treatment plans based on real-time and/or near real-time engagement and structured execution frameworks. Additionally, system 100 addresses the problem of delays in digital therapeutic application development due to the manual effort required to design, structure, and/or validate treatment plans. By leveraging APIs in a digital experience platform (e.g., Adobe XD, Sketch, Figma, InVision, Zeplin, and/or other platforms) integrating proprietary databases, and/or generating structured execution frameworks from natural language input, the system 100 significantly reduces the time and effort needed to construct and deploy a functional digital therapeutic application. Instead of manually coding at least one (e.g., each) treatment flow, a developer can provide a high-level natural language description of the treatment goals, and/or system 100 can generate the structured execution framework, compile skeleton code, and/or integrate it with existing UI components, allowing for rapid iteration and deployment of a large number of personalized digital therapeutic applications.

System 100 addresses the challenge of creating a generative AI-based platform individually tailored for a large number of users needing treatment across a range of physical, cognitive, social, and/or behavioral domains. The system 100 provides a structured execution framework for an application to dynamically adapt personalized treatment plans based on user progress and external data sources (e.g., HCP recommendation). For example, if a user reports high stress levels, the application can modify the execution path to prioritize relaxation exercises before cognitive restructuring interventions.

In some implementations, the system 100 can process natural language input to generate structured treatment plans, overcoming a significant hurdle in using NLP to describe a user journey within digital therapeutics. The traditional systems often struggle to translate unstructured user, administrator, or healthcare provider input into structured execution frameworks. The system 100 can apply at least one generative model to extract key therapeutic components, associate them with predefined actions, and/or generate a directed process graph that structures user interactions over time. For example, if an operator provides a natural language description of a treatment journey, the system 100 can identify necessary therapy modules, define conditional logic transitions based on user progress, and/or structure an adaptive digital therapeutic workflow.

In some implementations, the system 100 can interface with a centralized database 112 to retrieve, modify, and/or serve therapeutic content dynamically, overcoming a major hurdle in leveraging design centralization for digital therapeutic applications. The conventional therapeutic systems often require manual content structuring, leading to inefficiencies and inconsistencies in treatment plan implementation. The system 100 can integrate with external and internal repositories (e.g., Figma, proprietary databases) to systematically query, retrieve, and/or update UI components, treatment templates, and/or structured content assets. The system 100 can retrieve a structured module template from a digital experience platform (e.g., Adobe XD, Sketch, Figma, InVision, Zeplin, and/or other platforms), adapting its content based on an updated treatment framework.

In some implementations, the system 100 can translate natural language to a plan using generative AI. In some implementations, the plan can invoke a set of APIs that generates front-end JavaScript code (and/or any other markup-based code) and CSS code (and/or any other styling code), providing a digital therapeutic application that can be further polished by practitioners. The system 100 can generate a significant portion of the front-end code (e.g., UI) using proprietary APIs. The system 100 can inject a database 112 into a UI construction/digital experience platform (e.g., Adobe XD, Sketch, Figma, InVision, Zeplin, and/or other platforms) The system 100 can traverse a design structure generated by a database 112 to combine one or more structures into desired front-end code. The system 100 can construct a set of digital therapeutic components with improved accuracy by leveraging natural language input and proprietary APIs. The system 100 can provide for reduced timeline in application 118 function creation, automated UI component assembly, dynamic layout generation, predictable code quality in front-end interactions, consistent UI/UX, and/or any framework-aligned development workflow optimizations.

In some implementations, the natural language input can be for generating a treatment plan for a digital therapeutic application to address a plurality of conditions (e.g., neurological disorders and mental health conditions, metabolic syndromes and cardiovascular diseases, chronic pain conditions and rehabilitation protocols, and/or any modifiable health conditions using structured intervention).

### SYSTEMS AND METHODS FOR GENERATING A STRUCTURED EXECUTION FRAMEWORK IN A CONTENT GENERATION PIPELINE

With reference to FIG. 2, an example flow diagram illustrating a method for retrieving content from a database or generating content in a digital therapeutic pipeline and providing the content, in accordance with some implementations of the present disclosure. It should be understood that this and other implementations described herein are examples. Alternative configurations, elements (e.g., machines, interfaces, functions, orders, and/or groupings), and/or omissions are possible. Many elements are functional and can be implemented as discrete or distributed components, combined with others, and/or located in various configurations. Functions can be executed using hardware, firmware, and/or software, such as processors executing instructions stored in memory. The systems, methods, and/or processes can use components and functionality similar to the server system 400 and client computing system 414 of FIG. 4.

In FIG. 2, each block of method 200 represents a computing process that can be performed using hardware, firmware, and/or software, such as processors executing memory-stored instructions. The method can also be implemented as computer-readable instructions on storage media, provided as a standalone application, a service, a microservice via an API, and/or a plug-in. Method 200 is described with reference to the system of FIG. 1 but can also be executed by any other system or combination of systems described herein.

FIG. 2 is a flow diagram showing a method 200 for receiving, applying, identifying, causing, generating, administering, and/or providing operations, in accordance with some implementations of the present disclosure. Various operations of method 200 can relate to improving personalization and generation of a digital therapeutic application. Prior systems often rely on and/or use predefined content (e.g., treatment plans), which can lead to limited adaptability and reduced therapeutic impact. These technological problems can arise when these prior systems fail to provide dynamic and adequate treatment plans for use by an application, resulting in reduced engagement and ineffective outcomes. Method 200 of FIG. 2 can solve these technological problems by implementing AI-driven content generation personalized to each individual user, thereby improving application and downstream therapeutic efficacy.

The method 200, at block 210, includes receiving natural language input to retrieve from a database or generate content for a digital therapeutic application to address a condition. In some implementations, the processing circuits can receive natural language input to generate a treatment plan for a digital therapeutic application to address a condition(s). In some implementations, the natural language input can include instructions identifying treatment duration (e.g., one week, 30 days, three-month intervention, ongoing maintenance program, and/or other durations). In some implementations, the natural language input can include instructions identifying at least one treatment component to be performed by the user (e.g., guided meditation, exposure therapy, cognitive restructuring exercises, and/or other behavioral activation tasks). In some implementations, the natural language input can include instructions identifying at least one action (e.g., present breathing exercise module, prompt journaling reflection, adjust difficulty level of task, schedule follow-up session, and/or other actions) corresponding to at least one treatment component. In some implementations, the natural language input can include instructions identifying at least one criteria (e.g., successful completion of three consecutive sessions, self-reported stress reduction below threshold, engagement rate above 80%, adherence to prescribed intervention schedule, and/or other criteria) for completing the at least one treatment component. The input can be high-level treatment details (e.g., goals, duration, exercises, parameters for transitioning between exercises, and/or other details).

The method 200, at block 220, includes applying the natural language input and the condition and/or conditions (e.g., migraines) as input to at least one generative model to cause the at least one generative model to generate a directed process graph for the condition. In some implementations, the directed process graph can include a plurality of actions and conditional logic according to which to provide, for presentation, at least one of the plurality of actions. In some implementations, the one or more processing circuits can apply the natural language input and the condition and/or conditions as input to at least one generative model to cause the at least one generative model to generate a treatment plan for the condition. The treatment plan can include a list of treatment components and a schedule for presenting at least one treatment component of the list of treatment components. The treatment plan can include the schedule identifying a plurality of conditional steps corresponding with at least one of the list of treatment components of a treatment journey. Additionally, each of the plurality of conditional steps can correspond with a conditional logic to satisfy.

In some implementations, the schedule of the treatment plan can include a plurality of conditional logic corresponding with at least one treatment component and initiating at least one subsequent treatment component. The at least one subsequent treatment component can be provided based at least on a completion of or failure of at least one prior treatment component or a completion of or a failure to meet predefined criteria for the at least one prior treatment component. In some implementations, the directed process graph can include a hierarchical arrangement of treatment components representing the plurality of actions to be performed by the user, the hierarchical arrangement arranged based on the plurality of timing parameters identifying a sequence and duration corresponding with at least one of the plurality of actions. In some implementations, the directed process graph can include a plurality of execution paths corresponding to a plurality of treatment workflows of a treatment journey. Additionally, at least one (e.g., each) treatment workflow can include a sequence of therapeutic actions for the user to perform to address or manage the condition (e.g., obesity).

In some implementations, the at least one generative model can be a deep learning model, a supervised learning model, and/or an unsupervised learning model. Additionally, causing the at least one generative model to generate the directed process graph can include processing the natural language input to extract a plurality of actionable elements, associating the plurality of actionable elements with the plurality of actions and a plurality of conditional logic, and/or arranging the plurality of actions and the plurality of conditional logic into a hierarchical structure. The conditional logic of the directed process graph can correspond with at least one treatment component and initiating at least one subsequent treatment component. The at least one subsequent treatment component is provided based at least on a completion of or failure of at least one prior treatment component or a completion of or a failure to meet predefined criteria for the at least one prior treatment component.

The method 200, at block 230, can identify at least one data object. The processing circuits can identify data objects via an interface of a database. In some implementations, a data object can correspond with at least one of the plurality of actions included in the directed process graph. In some implementations, the processing circuits can retrieve, via an interface of the database, a plurality of content items from a database. The plurality of content items can correspond with at least one treatment component of the list of treatment components in the treatment plan. In some implementations, identifying the at least one data object includes querying the database maintaining the at least one data object. Querying the database can include obtaining, using at least one API request, the at least one data object corresponding to at least one therapeutic action or configuration.

The method 200, at block 240, includes generating a structured execution framework. The processing circuits can generate a structured execution framework using at least one data object and the directed process graph. The structured execution framework can identify a plurality of content items identifying at least one of the plurality of actions. Additionally, the structured execution framework can identify corresponding conditional logic according to which to provide, for presentation, at least one of the plurality of actions. In some implementations, the processing circuits can arrange, using the plurality of content items and the schedule, the list of treatment components of the treatment plan into a structured data package for presentation in the digital therapeutic application.

In some implementations, the processing circuits can generate, using the structured data package, a plurality of instructions configured to cause the digital therapeutic application to present at least one of the plurality of content items according to the arrangement of the list of treatment components of the treatment plan. The structured data package can correspond to a mapping of the plurality of content items, the schedule, and/or a plurality of execution dependencies of the structured data package. Additionally, the processing circuits can generate, using the structured execution framework, a plurality of instructions configured to cause the digital therapeutic application to present the content including at least one of the plurality of content items according to the directed process graph. The structured execution framework corresponds to a mapping of the plurality of content items, the corresponding conditional logic, a plurality of timing parameters, and/or a plurality of execution dependencies of the directed process graph.

In some implementations, the processing circuits can emulate, using an emulator, performance of the plurality of actions based on processing at least a portion of the plurality of instructions according to the structured execution framework. The plurality of instructions can include at least one operation for performing at least one of the plurality of actions corresponding with at least one timing parameter. In some implementations, the processing circuits can compile skeleton code retrieved from at least one database or generate, using the structured execution framework and the directed process graph. The skeleton code can include at least one programmatic construct corresponding with the plurality of actions and corresponding conditional logic. In some implementations, the processing circuits can compile skeleton code retrieved from at least one database or generate, using the structured data package, the skeleton code including at least one programmatic construct corresponding with the list of treatment components of the treatment plan and the schedule.

In some implementations, the processing circuits can provide the treatment plan or the skeleton code to an administrator for review. Additionally, the processing circuits can receive at least one update to the treatment plan or the skeleton code prior to providing the structured data package or using the skeleton code in the digital therapeutic application. The processing circuits can receive updates from an administrator or HCP or other external source.

In some implementations, at least one of the plurality of actions corresponds to a treatment component or a response to a diagnostic or clinical scale/questionnaire query. In some implementations, the processing circuits can in response to a completion of the treatment component or providing the response to the diagnostic or clinical scale/questionnaire query, update an activity record to include a completion status, timestamp data, and/or data generated or obtained during the treatment component or diagnostic or clinical scale/questionnaire query.

The method 200, at block 250, includes providing the structured execution framework. The processing circuits can provide the structured data package. The processing circuits can provide the structured execution framework to an administrator for review. Additionally, the processing circuits can receive an update to the structured execution framework. The processing circuits can provide the structured execution framework or skeleton code to a digital therapeutic application for use.

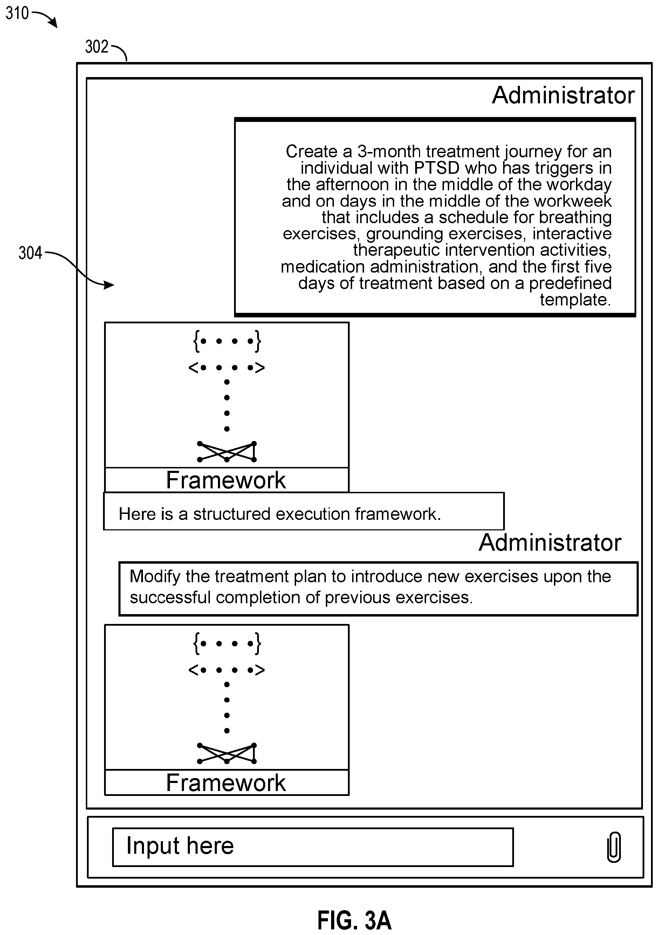

Referring now to FIG. 3A is an example interface for providing receiving natural language input to retrieve content from a database or generate content for a digital therapeutic application to address a condition (e.g., nausea), in accordance with some implementations of the present disclosure. The system 100 interfaces with a device 302 of an administrator, digital therapeutic application user, and/or other source via an interface to generate a structured execution framework. The system 100 can provide an interface 304 for model interaction 310. In this scenario, the model(s) (e.g., the model(s) 108 and/or the model(s) of design system 114) are configured to process natural language input and generate a structured execution framework dynamically. The model interaction 310 involves the administrator inputting instructions for the model(s) 108 to create a treatment journey. The administrator begins, "create a 3-month treatment journey for an individual with PTSD who has triggers in the afternoon in the middle of the workday and on days in the middle of the workweek that includes a schedule for breathing exercises, grounding exercises, interactive therapeutic intervention activities, medication administration, and the first five days of treatment based on a predefined template." The model(s) 108 and/or the model(s) of design system 114 generate the reply of the chatbot: "Here is a structured execution framework," including an attached structured execution framework. In the background, the processing circuitry of system 100 processes the input via the model(s) 108 to generate a directed process graph including a plurality of actions and conditional logic. The processing circuitry can use the directed process graph to generate the structured execution framework. The administrator responds: "Modify the treatment plan to introduce new exercises upon the successful completion of 80% of the previous exercises." Metrics such as input clarity and scenario-specific objectives can be tracked to personalize interactions and facilitate dynamic framework generation and adaptive responses.

The structured execution framework can be a structured representation of the treatment journey generated from the natural language input, including a plurality of actions, conditional logic, and/or timing attributes organized to support generating executable instructions for constructing a digital therapeutic application. The structured execution framework can be JavaScript configuration file containing structured JSON data that defines treatment session flows, conditional triggers, and/or interactive components. The JavaScript file can instantiate dynamic UI components, register event listeners to track user interactions, and/or execute conditional logic that determines content sequencing based on user progress. For instance, a treatment plan can adjust its execution path in real-time and/or near real-time by evaluating engagement metrics and transitioning users to appropriate therapeutic modules accordingly. The structured execution framework can be a schema-driven representation using an API of a design tool to query for all existing modules and submodules in the digital experience platform space. The system can retrieve predefined UI components, such as treatment session layouts, button interactions, and/or progress indicators, integrating them into a structured execution framework. A database, and/or an orchestration tool, can compile these UI elements into a finalized digital therapeutic application. The compiled UI and execution logic can be deployed in an emulator for testing, allowing administrators to visualize content transitions, validate user interactions, and/or refine the overall treatment experience before deployment. The structured execution framework can include a drag- and-drop file containing CSS code shells to be imported into a front-end development environment, where predefined style components are automatically mapped to corresponding UI elements in the digital therapeutic application. When a developer drags and drops the file into a web-based editor or IDE, the system can parse the CSS structure, apply class-based styling rules, and/or generate a responsive layout that dynamically adjusts to different screen sizes and accessibility settings.

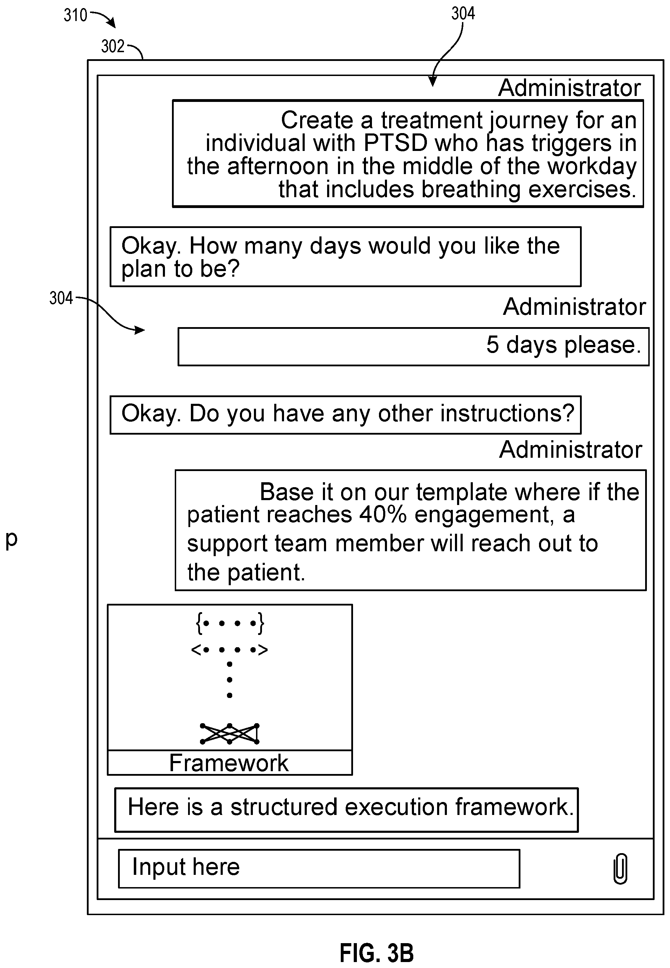

Referring now to FIG. 3B is an example interface for providing a framework, in accordance with some implementations of the present disclosure. The system 100 interfaces with a device 302 of an administrator, digital therapeutic application user, and/or other source via an interface to generate a structured execution framework. The system 100 can provide an interface 304 for model interaction 310. The model(s) 108 can generate prompts and display messages for the administrator to provide additional information used to create a treatment journey. The model(s) 108 can generate prompts and display additional messages in response to administrator input. The additional interaction between the model(s) 108 and administrator can provide for additional input into the model(s) to generate a treatment plan.

In this scenario, the model(s) 108 are configured to process natural language input and generate a structured execution framework dynamically. The model interaction 310 involves the administrator inputting instructions for the model(s) 108 to create a treatment journey. The administrator begins, "create a treatment journey for an individual with PTSD who has triggers in the afternoon in the middle of the workday and on days in the middle of the workweek that includes a schedule for breathing exercises." The model(s) 108 generate the reply of the chatbot: "Okay. How many days would you like the plan to be?" The administrator responds, "5 days please." The system 100 applies the model(s) 108 and/or model(s) of design system 114 to process this input and generate the response: "Okay. Do you have any other instructions?" The administrator continues, "Base it on our template where if the user reaches 40% engagement, a support team member will reach out to the user." The system 100 applies the model(s) 108 and/or model(s) of design system 114 to process this input and generate the response: "Here is a structured execution framework.", including an attached structured execution framework.

The system 100 can apply the model(s) 108 and/or model(s) of design system 114 to generate a structured execution framework from other natural language prompts. As shown, the system 100 can apply the model(s) 108 to generate prompts for the administrator to provide input. The system 100 can apply the model(s) 108 to interact with the administrator to provide prompts regarding duration, exercise types, treatment difficulty levels, conditional logic branching, user progress tracking methods, adaptive intervention adjustments, and/or other prompts. The system 100 can apply the model(s) 108 to generate a process graph based on the interactions. Additionally, the system 100 generating additional prompts for administrator input can allow the process graph for the treatment plan to be dynamically updated by incorporating newly provided details (e.g., an updated HCP recommendation). If the administrator modifies the treatment duration, adds new therapeutic activities, and/or specifies additional conditional transitions, the system 100 can regenerate the directed process graph to reflect these changes for a continuously refined treatment workflow, where at least one (e.g., each) modification can be incorporated into the execution framework.

The system 100 can generate feedback for conditional logic represented between process graph modules to further create a directed graph of user interaction flow for any amount of time (e.g., days, weeks, months, years). In some implementations, an administrator can review the model(s) 108 output to design a final treatment journey.

Various operations described herein can be implemented on computer systems. FIG. 4 shows a simplified block diagram of a representative server system 400, user computer system 414, and/or network 426 usable to implement certain embodiments of the present disclosure. In various embodiments, server system 400 or similar systems can implement services or servers described herein or portions thereof. System 100 described herein can be like the server system 400. Server system 400 can have a modular design that incorporates a number of modules 402 (e.g., blades in a blade server embodiment); while two modules 402 are shown, any number can be provided. At least one (e.g., each) module 402 can include processing unit(s) 404 and local storage 406.

Processing unit(s) 404 can include a single processor, which can have one or more cores, and/or multiple processors. In some embodiments, processing unit(s) 404 can include a general-purpose primary processor as well as one or more special-purpose co-processors, such as graphics processors, digital signal processors, and/or the like. In some embodiments, some or all processing units 404 can be implemented using customized circuits, such as application specific integrated circuits (ASICs) or field programmable gate arrays (FPGAs). In some embodiments, such integrated circuits execute instructions that are stored on the circuit itself. In other embodiments, processing unit(s) 404 can execute instructions stored in local storage 406. Any type of processors in any combination can be included in processing unit(s) 404.

Local storage 406 can include volatile storage media (e.g., DRAM, SRAM, SDRAM, and/or the like) and/or non-volatile storage media (e.g., magnetic, and/or optical disk, flash memory, and/or the like). Storage media incorporated in local storage 406 can be fixed, removable, and/or upgradeable as desired. Local storage 406 can be physically or logically divided into various subunits such as a system memory, a read-only memory (ROM), and/or a permanent storage device. The system memory can be a read-and-write memory device or a volatile read-and-write memory, such as dynamic random-access memory. The system memory can store some or all of the instructions and data that processing unit(s) 404 need at runtime. The ROM can store static data and instructions that are needed by processing unit(s) 404. The permanent storage device can be a non-volatile read-and-write memory device that can store instructions and data even when module 402 is powered down. The term "storage medium" as used herein includes any medium in which data can be stored indefinitely (subject to overwriting, electrical disturbance, power loss, and/or the like) and does not include carrier waves and transitory electronic signals propagating wirelessly or over wired connections.

In some embodiments, local storage 406 can store one or more software programs to be executed by processing unit(s) 404, such as an operating system and/or programs implementing various server functions such as functions of the system 100 or any other system described herein, and/or any other server(s) associated with system 100 or any other system described herein.

"Software" refers generally to sequences of instructions that, when executed by processing unit(s) 404, cause server system 400 (or portions thereof) to perform various operations, thus defining one or more specific machine embodiments that execute and perform the operations of the software programs. The instructions can be stored as firmware residing in read-only memory and/or program code stored in non-volatile storage media that can be read into volatile working memory for execution by processing unit(s) 404. Software can be implemented as a single program or a collection of separate programs or program modules that interact as desired. From local storage 406 (or non-local storage described below), processing unit(s) 404 can retrieve program instructions to execute and data to process to execute various operations described above.

In some server systems 400, multiple modules 402 can be interconnected via a bus or other interconnect 408, forming a local area network (LAN) that supports communication between modules 402 and other components of server system 400. Interconnect 408 can be implemented using various technologies, including server racks, hubs, routers, etc.

A wide-area network (WAN) interface 410 can provide data communication capability between the LAN (e.g., through the interconnect 408) and the network 426, such as the Internet. Other technologies can be used to communicatively couple the server system with the network 426, including wired (e.g., Ethernet, IEEE 802.3 standards) and/or wireless technologies (e.g., Wi-Fi, IEEE 802.11 standards).

In some embodiments, local storage 406 is intended to provide working memory for processing unit(s) 404, providing fast access to programs and/or data to be processed while reducing traffic on interconnect 408. Storage for larger quantities of data can be provided on the LAN by one or more mass storage subsystems 412 that can be connected to interconnect 408. Mass storage subsystem 412 can be based on magnetic, optical, semiconductor, and/or other data storage media. Direct attached storage, storage area networks, network-attached storage, and/or the like can be used. Any data stores or other collections of data described herein as being produced, consumed, and/or maintained by a service or server can be stored in mass storage subsystem 412. In some embodiments, additional data storage resources can be accessible via WAN interface 410 (potentially with increased latency).

Server system 400 can operate in response to requests received via WAN interface 410. At least one of modules 402 can implement a supervisory function and assign discrete tasks to other modules 402 in response to received requests. Work allocation techniques can be used. As requests are processed, results can be returned to the requester via WAN interface 410. Such operation can generally be automated. Further, in some embodiments, WAN interface 410 can connect multiple server systems 400 to at least one (e.g., each) other, providing scalable systems capable of managing high volumes of activity. Other techniques for managing server systems and server farms (collections of server systems that cooperate) can be used, including dynamic resource allocation and reallocation.

Server system 400 can interact with various user-owned or user-operated devices via a WAN such as the Internet. An example of a user-operated device is shown in FIG. 4 as user computing system 414. User computing system 414 can be implemented as a consumer device such as a smartphone, other mobile phone, tablet computer, wearable user device (e.g., smart watch, eyeglasses), desktop computer, laptop computer, and/or so on. The user computing system 414 can communicate via WAN interface 410. User computing system 414 can include computer components such as processing unit(s) 416, storage device 418, network interface 420, user input device 422, and/or user output device 424. User computing system 414 can be a user device implemented in a variety of form factors, such as a desktop computer, laptop computer, tablet computer, smartphone, other mobile user device, wearable user device, and/or the like.

Processing unit(s) 416 and storage device 418 can be similar to processing unit(s) 404 and local storage 406 described above. Suitable devices can be selected based on the demands to be placed on user computing system 414; user computing system 414 can be implemented as a "thin" user with limited processing capability or as a high-powered user device. User computing system 414 can be provisioned with program code executable by processing unit(s) 416 to allow various interactions with server system 400.

Network interface 420 can provide a connection to the network 426, such as a WAN (e.g., the Internet) to which WAN interface 410 of server system 400 is also connected. In various embodiments, network interface 420 can include a wired interface (e.g., Ethernet) and/or a wireless interface implementing various RF data communication standards such as Wi-Fi, Bluetooth, and/or cellular data network standards (e.g., 3G, 4G, LTE, 5G, etc.).

User input device 422 can include any device (or devices) via which a user can provide signals to user computing system 414; user computing system 414 can interpret the signals as indicative of user requests or information. In various embodiments, user input device 422 can include at least one of a keyboard, touch pad, touch screen, mouse, and/or other pointing device, scroll wheel, click wheel, dial, button, switch, keypad, microphone, and/or so on.

User output device 424 can include any device via which user computing system 414 can provide information to a user. The user output device 424 can include display-to-display images generated by or delivered to user computing system 414. The display can incorporate various image generation technologies, e.g., a liquid crystal display (LCD), light-emitting diode (LED) display including organic light-emitting diodes (OLED), projection system, cathode ray tube (CRT), and/or the like, together with supporting electronics (e.g., digital-to-analog or analog-to-digital converters, signal processors, and/or the like). Some embodiments can include a device such as a touchscreen that function as both input and output device. In some embodiments, other user output devices 424 can be provided in addition to or instead of a display. Examples include indicator lights, speakers, tactile "display" devices, printers, and/or so on.

Some embodiments include electronic components, such as microprocessors, storage, and/or memory that store computer program instructions in a computer readable storage medium. Many of the features described in this specification can be implemented as processes that are specified as a set of program instructions encoded on a computer readable storage medium. When one or more processing units execute these program instructions, they cause the processing unit(s) to perform various operations indicated in the program instructions. Examples of program instructions or computer code include machine code, such as is produced by a compiler, and/or files including higher-level code that are executed by a computer, an electronic component, and/or a microprocessor using an interpreter. Through suitable programming, processing unit(s) 404 and 416 can provide various functionality for server system 400 and user computing system 414, including any of the functionality described herein as being performed by a server or user, and/or other functionality.

It will be appreciated that server system 400 and user computing system 414 are illustrative and that variations and modifications are possible. Computer systems used in connection with embodiments of the present disclosure can have other capabilities not specifically described here. Further, while server system 400 and user computing system 414 are described with reference to particular blocks, it is to be understood that these blocks are defined for convenience of description and are not intended to imply a particular physical arrangement of component parts. For instance, different blocks can be but need not be in the same facility, in the same server rack, and/or on the same motherboard. Further, the blocks need not correspond to physically distinct components. Blocks can be configured to perform various operations, e.g., by programming a processor or providing appropriate control circuitry, and/or various blocks can or cannot be reconfigurable depending on how the initial configuration is obtained. Embodiments of the present disclosure can be realized in a variety of apparatus including electronic devices implemented using any combination of circuitry and software.

While the disclosure has been described with respect to specific embodiments, one skilled in the art will recognize that numerous modifications are possible. Embodiments of the disclosure can be realized using a variety of computer systems and communication technologies, including, but not limited to, specific examples described herein. Embodiments of the present disclosure can be realized using any combination of dedicated components and/or programmable processors and/or other programmable devices. The various processes described herein can be implemented on the same processor or different processors in any combination. Where components are described as being configured to perform certain operations, such configuration can be accomplished, e.g., by designing electronic circuits to perform the operation, by programming programmable electronic circuits (such as microprocessors) to perform the operation, and/or any combination thereof. Further, while the embodiments described above can refer to specific hardware and software components, those skilled in the art will appreciate that different combinations of hardware and/or software components can also be used and that particular operations described as being implemented in hardware can also be implemented in software or vice versa.

Computer programs incorporating various features of the present disclosure can be encoded and stored on various computer readable storage media; suitable media include magnetic disk or tape, optical storage media such as compact disk (CD) or digital versatile disk (DVD), flash memory, and/or other non-transitory media. Computer readable media encoded with the program code can be packaged with a compatible electronic device, and/or the program code can be provided separately from electronic devices (e.g., via Internet download or as a separately packaged computer-readable storage medium).

Aspects of the disclosure are set out in the following numbered clauses, in which:
1. A method for generating a structured treatment plan and integrating content in a digital therapeutic application, the method comprising:
   receiving, by one or more processors, natural language input to generate a treatment plan for a digital therapeutic application to address a condition;
   applying, by the one or more processors, the natural language input and the condition as input to at least one generative model to cause the at least one generative model to generate a treatment plan for the condition, the treatment plan comprising a list of treatment components and a schedule for presenting at least one treatment component of the list of treatment components;
   retrieving, by the one or more processors, from a database, a plurality of content items corresponding with at least one treatment component of the list of treatment components in the treatment plan;
   arranging, by the one or more processors, using the plurality of content items and the schedule, the list of treatment components of the treatment plan into a structured data package for presentation in the digital therapeutic application; and
   providing, by the one or more processors, the structured data package.
2. The method of clause 1, further comprising generating, by the one or more processors, using the structured data package, a plurality of instructions configured to cause the digital therapeutic application to present at least one of the plurality of content items according to the arrangement of the list of treatment components of the treatment plan.
3. The method of clause 1 or clause 2, wherein the structured data package corresponds to a mapping of the plurality of content items, the schedule, or a plurality of execution dependencies of the structured data package.
4. The method of any preceding clause, wherein the treatment plan comprises the schedule identifying a plurality of conditional steps corresponding with at least one of the list of treatment components of a treatment journey.
5. The method of any preceding clause, wherein the schedule of the treatment plan comprises a plurality of conditional logic corresponding with at least one treatment component and initiating at least one subsequent treatment component, and wherein the at least one subsequent treatment component is provided based at least on a completion of or failure of at least one prior treatment component or a completion of or a failure to meet predefined criteria for the at least one prior treatment component.
6. The method of any preceding clause, wherein the natural language input comprises instructions identifying at least one of (i) a treatment duration, (ii) at least one treatment component, (iii) at least one action corresponding to the at least one treatment component, or (iv) at least one criteria for completing the at least one treatment component.
7. The method of any preceding clause, further comprising compiling, by the one or more processors, skeleton code retrieved from at least one database or generate, using the structured data package, the skeleton code comprising at least one programmatic construct corresponding with the list of treatment components of the treatment plan and the schedule.
8. The method of clause 7, further comprising:
   providing, by the one or more processors, the treatment plan or the skeleton code to an administrator for review; and
   receiving, by the one or more processors, from the administrator, at least one update to the treatment plan or the skeleton code prior to providing the structured data package or using the skeleton code in the digital therapeutic application.
9. A computer-readable storage medium having computer-readable instructions stored thereon that, when executed by one or more processors, cause the one or more processors to perform the method of any preceding clause.
10. A system for structured treatment plan generation and content integration in a digital therapeutic application, comprising:
   one or more processors coupled with memory configured to:
   receive natural language input to generate a treatment plan for a digital therapeutic application to address a condition;
   apply the natural language input and the condition as input to at least one generative model to cause the at least one generative model to generate a treatment plan for the condition, the treatment plan comprising a list of treatment components and a schedule for presenting at least one treatment component of the list of treatment components;
   retrieve, from a database, a plurality of content items corresponding with at least one treatment component of the list of treatment components in the treatment plan;
   arrange, using the plurality of content items and the schedule, the list of treatment components of the treatment plan into a structured data package for presentation in the digital therapeutic application; and
   provide the structured data package.
11. The system of clause 10, wherein the one or more processors are further configured to:
   generate, using the structured data package, a plurality of instructions configured to cause the digital therapeutic application to present at least one of the plurality of content items according to the arrangement of the list of treatment components of the treatment plan.
12. The system of clause 10 or clause 11, wherein the structured data package corresponds to a mapping of the plurality of content items, the schedule, or a plurality of execution dependencies of the structured data package.
13. The system of any of clauses 10 to 12, wherein the treatment plan comprises the schedule identifying a plurality of conditional steps corresponding with at least one of the list of treatment components of a treatment journey.
14. The system of any of clauses 10 to 13, wherein the schedule of the treatment plan comprises a plurality of conditional logic corresponding with at least one treatment component and initiating at least one subsequent treatment component, and wherein the at least one subsequent treatment component is provided based at least on a completion of or failure of at least one prior treatment component or a completion of or a failure to meet predefined criteria for the at least one prior treatment component.
15. The system of any of clauses 10 to 14, wherein the natural language input comprises instructions identifying at least one of (i) a treatment duration, (ii) at least one treatment component, (iii) at least one action corresponding to the at least one treatment component, or (iv) at least one criteria for completing the at least one treatment component.
16. The system of any of clauses 10 to 15, wherein the one or more processors are further configured to:
   compile skeleton code retrieved from at least one database or generate, using the structured data package, the skeleton code comprising at least one programmatic construct corresponding with the list of treatment components of the treatment plan and the schedule.
17. The system of clause 16, wherein the one or more processors are further configured to:
   provide the treatment plan or the skeleton code to an administrator for review; and
   receive, from the administrator, at least one update to the treatment plan or the skeleton code prior to providing the structured data package or using the skeleton code in the digital therapeutic application.
18. A system, comprising:
   one or more processors coupled with memory configured to:
   receive natural language input to generate content for a digital therapeutic application to address a condition;
   apply the natural language input and the condition as input to at least one generative model to cause the at least one generative model to generate a directed process graph for the condition, the directed process graph comprising a plurality of actions and conditional logic according to which to provide, for presentation, at least one of the plurality of actions;
   identify, at least one data object corresponding with at least one of the plurality of actions comprised in the directed process graph;
   generate, using the at least one data object and the directed process graph, a structured execution framework identifying (i) a plurality of content items identifying at least one of the plurality of actions and (ii) corresponding conditional logic according to which to provide, for presentation, at least one of the plurality of actions; and
   provide the structured execution framework.
19. The system of clause 18, wherein the one or more processors are further configured to:
   generate, using the structured execution framework, a plurality of instructions configured to cause the digital therapeutic application to present the content comprising at least one of the plurality of content items according to the directed process graph.
20. The system of clause 19, wherein the structured execution framework corresponds to a mapping of the plurality of content items, the corresponding conditional logic, a plurality of timing parameters, or a plurality of execution dependencies of the directed process graph.
21. The system of clause 19 or clause 20, wherein the one or more processors are further configured to:
   emulate, using an emulator, performance of the plurality of actions based on processing at least a portion of the plurality of instructions according to the structured execution framework, and wherein the plurality of instructions comprises at least one operation for performing at least one of the plurality of actions corresponding with at least one timing parameter.
22. The system of clause 20, wherein the directed process graph comprises a hierarchical arrangement of components representing the plurality of actions, the hierarchical arrangement arranged based on the plurality of timing parameters identifying a sequence and duration corresponding with at least one of the plurality of actions.
23. The system of clause 22, wherein the at least one generative model is at least one of (i) a deep learning model, (ii) a supervised learning model, or (iii) an unsupervised learning model, and wherein causing the at least one generative model to generate the directed process graph comprises processing the natural language input to extract a plurality of actionable elements, associating the plurality of actionable elements with the plurality of actions and a plurality of conditional logic, and arranging the plurality of actions and the plurality of conditional logic into a hierarchical structure.
24. The system of any one of clauses 18 to 23, wherein the conditional logic of the directed process graph comprises a plurality of conditional logic corresponding with at least one treatment component and initiating at least one subsequent treatment component, and wherein the at least one subsequent treatment component is provided based at least on a completion of or failure of at least one prior treatment component or a completion of or a failure to meet predefined criteria for the at least one prior treatment component.
25. The system of any one of clauses 18 to 24, wherein the natural language input comprises instructions identifying at least one of (i) a treatment duration, (ii) at least one treatment component, (iii) at least one action corresponding to the at least one treatment component, or (iv) at least one criteria for completing the at least one treatment component.
26. The system of clause 25, wherein the directed process graph comprises a plurality of execution paths, and wherein the plurality of execution paths of the directed process graph correspond to a plurality of treatment workflows of a treatment journey, wherein each treatment workflow of the plurality of treatment workflows comprises a sequence of therapeutic actions to address or manage the condition.
27. The system of any one of clauses 18 to 26, wherein at least one of the plurality of actions corresponds to a treatment component or a response to a diagnostic query, and wherein the one or more processors are further configured to:
   in response to a completion of the treatment component or providing the response to the diagnostic query, update an activity record to comprise a completion status, timestamp data, or data generated or obtained during the treatment component or diagnostic query.
28. The system of any one of clauses 18 to 27, wherein the one or more processors are further configured to:
   compile skeleton code retrieved from at least one database or generate, using the structured execution framework and the directed process graph, the skeleton code comprising at least one programmatic construct corresponding with the plurality of actions and corresponding conditional logic.
29. The system of clause 28, wherein the one or more processors are further configured to:
   provide the directed process graph or the skeleton code to an administrator for review; and
   receive, from the administrator, at least one update to the directed process graph or the skeleton code prior to providing the structured execution framework or using the skeleton code in the digital therapeutic application.
30. The system of any one of clauses 18 to 29, wherein identifying the at least one data object comprises querying a database maintaining the at least one data object, and wherein querying the database comprises obtaining, using at least one application programming interface (API) request, the at least one data object corresponding to at least one therapeutic action or configuration.
31. The system of clause 30, wherein the at least one data object comprises at least one of the plurality of content items and corresponding parameters, and wherein the corresponding parameters comprise at least one of a content item relationship, a timing parameter, or metadata for representing the at least one therapeutic action as a node or relationship in the directed process graph.
32. A method, comprising:
   receiving, by one or more processors, natural language input to generate content for a digital therapeutic application to address a condition;
   applying, by the one or more processors, the natural language input and the condition as input to at least one generative model to cause the at least one generative model to generate a directed process graph for the condition, the directed process graph comprising a plurality of actions and conditional logic according to which to provide, for presentation, at least one of the plurality of actions;
   identifying, by the one or more processors, at least one data object corresponding with at least one of the plurality of actions comprised in the directed process graph;
   generating, by the one or more processors using the at least one data object and the directed process graph, a structured execution framework identifying (i) a plurality of content items identifying at least one of the plurality of actions and (ii) corresponding conditional logic according to which to provide, for presentation, at least one of the plurality of actions; and
   providing, by the one or more processors, the structured execution framework.
33. The method of clause 32, further comprising:
   generating, by the one or more processors using the structured execution framework, a plurality of instructions configured to cause the digital therapeutic application to present the content comprising at least one of the plurality of content items according to the directed process graph.
34. The method of clause 33, wherein the structured execution framework corresponds to a mapping of the plurality of content items, the corresponding conditional logic, a plurality of timing parameters, or a plurality of execution dependencies of the directed process graph.
35. The method of clause 33 or clause 34, further comprising:
   emulating, by one or more processors using an emulator, performance of the plurality of actions based on processing at least a portion of the plurality of instructions according to the structured execution framework, and wherein the plurality of instructions comprises at least one operation for performing at least one of the plurality of actions corresponding with at least one timing parameter.
36. The method of clause 35, wherein the at least one generative model is at least one of (i) a deep learning model, (ii) a supervised learning model, or (iii) an unsupervised learning model, and wherein causing the at least one generative model to generate the directed process graph comprises processing the natural language input to extract a plurality of actionable elements, associating the plurality of actionable elements with the plurality of actions and a plurality of conditional logic, and arranging the plurality of actions and the plurality of conditional logic into a hierarchical structure.
37. The method of any one of clauses 32 to 36, wherein the conditional logic of the directed process graph comprises a plurality of conditional logic corresponding with at least one treatment component and initiating at least one subsequent treatment component, and wherein the at least one subsequent treatment component is provided based at least on a completion of or failure of at least one prior treatment component or a completion of or a failure to meet predefined criteria for the at least one prior treatment component.
38. The method of any one of clauses 32 to 37, wherein the natural language input comprises instructions identifying at least one of (i) a treatment duration, (ii) at least one treatment component, (iii) at least one action corresponding to the at least one treatment component, or (iv) at least one criteria for completing the at least one treatment component.
39. The method of clause 38, wherein the directed process graph comprises a plurality of execution paths, and wherein the plurality of execution paths of the directed process graph correspond to a plurality of treatment workflows of a treatment journey, wherein each treatment workflow of the plurality of treatment workflows comprises a sequence of treatment components to address or manage the condition.

Thus, although the disclosure has been described with respect to specific embodiments, it will be appreciated that the disclosure is intended to cover all modifications and equivalents within the scope of the following claims.

## Claims

1. A method for generating a structured treatment plan and integrating content in a digital therapeutic application, the method comprising:
receiving, by one or more processors, natural language input to generate a treatment plan for a digital therapeutic application to address a condition;
applying, by the one or more processors, the natural language input and the condition as input to at least one generative model to cause the at least one generative model to generate a treatment plan for the condition, the treatment plan comprising a list of treatment components and a schedule for presenting at least one treatment component of the list of treatment components;
retrieving, by the one or more processors, from a database, a plurality of content items corresponding with at least one treatment component of the list of treatment components in the treatment plan;
arranging, by the one or more processors, using the plurality of content items and the schedule, the list of treatment components of the treatment plan into a structured data package for presentation in the digital therapeutic application; and
providing, by the one or more processors, the structured data package.

2. The method of claim 1, further comprising generating, by the one or more processors, using the structured data package, a plurality of instructions configured to cause the digital therapeutic application to present at least one of the plurality of content items according to the arrangement of the list of treatment components of the treatment plan.

3. The method of claim 1 or claim 2, wherein the structured data package corresponds to a mapping of the plurality of content items, the schedule, or a plurality of execution dependencies of the structured data package.

4. The method of any preceding claim, wherein the treatment plan comprises the schedule identifying a plurality of conditional steps corresponding with at least one of the list of treatment components of a treatment journey.

5. The method of any preceding claim, wherein the schedule of the treatment plan comprises a plurality of conditional logic corresponding with at least one treatment component and initiating at least one subsequent treatment component, and wherein the at least one subsequent treatment component is provided based at least on a completion of or failure of at least one prior treatment component or a completion of or a failure to meet predefined criteria for the at least one prior treatment component.

6. The method of any preceding claim, wherein the natural language input comprises instructions identifying at least one of (i) a treatment duration, (ii) at least one treatment component, (iii) at least one action corresponding to the at least one treatment component, or (iv) at least one criteria for completing the at least one treatment component.

7. The method of any preceding claim, further comprising compiling, by the one or more processors, skeleton code retrieved from at least one database or generate, using the structured data package, the skeleton code comprising at least one programmatic construct corresponding with the list of treatment components of the treatment plan and the schedule.

8. The method of claim 7, further comprising:
providing, by the one or more processors, the treatment plan or the skeleton code to an administrator for review; and
receiving, by the one or more processors, from the administrator, at least one update to the treatment plan or the skeleton code prior to providing the structured data package or using the skeleton code in the digital therapeutic application.

9. A computer-readable storage medium having computer-readable instructions stored thereon that, when executed by one or more processors, cause the one or more processors to perform the method of any preceding claim.

10. A system for structured treatment plan generation and content integration in a digital therapeutic application, comprising:
one or more processors coupled with memory configured to perform the method of any of claims 1 to 8.
